# EUROPEAN PATENT SPECIFICATION

(11) **EP 4 456 963 B1**
(45) Date of publication and mention of the grant of the patent: **09.09.2026**
(21) Application number: 22917394.3
(22) Date of filing: 30.12.2022
(51) Int. Cl.: A61M 37/00, A61M 5/46

(54) **SYSTEM FOR USE IN THE APPLICATION AND REMOVAL OF TATTOOS AND OTHER SKIN TREATMENTS**
SYSTEM ZUR VERWENDUNG BEI DER ANWENDUNG UND ENTFERNUNG VON TÄTOWIERUNGEN UND ANDEREN HAUTBEHANDLUNGEN
SYSTÈME DESTINÉ À ÊTRE UTILISÉ DANS L'APPLICATION ET LE RETRAIT DE TATOUAGES ET D'AUTRES TRAITEMENTS DE LA PEAU

(30) Priority: 31.12.2021 US 202163295610 P; 19.10.2022 US 202263417466 P
(43) Date of publication of application: 06.11.2024
(73) Proprietor: Rejuvatek Medical, Inc., Sandy, UT 84093 (US)
(72) Inventor: NIVEN, Gregg, D., Kaysville, UT 84037 (US); TURNER, Timothy, N., West Jordan, UT 84084 (US); CALACINO, Pier, F., Sandy, UT 84092 (US); SAVAGE, Jack, H., Sandy, UT 84093 (US)
(74) Representative: Lewis Silkin LLP
(86) International application number: PCT/US2022/054367
(87) International publication number: WO 2023/129729

(56) References cited:
- CN-B- 101 897 602
- US-A1- 2006 041 241
- US-A1- 2007 156 095
- US-A1- 2008 247 637
- US-A1- 2013 123 746
- US-A1- 2017 065 807
- US-A1- 2020 138 467
- US-A1- 2021 244 928
- US-A1- 2021 386 987
- US-B2- 10 702 684
- US-B2- 9 005 158

## Description

### FIELD OF THE INVENTION

The present invention relates generally to the application and removal of tattoos and, in particular, to the use of a reciprocating, rotating needle assembly.

### CROSS-REFERENCE TO RELATED APPLICATIONS

This application claims priority to U.S. Provisional Application No. 63/295,610 filed on 31 December 2021 and U.S. Provisional Application No. 63/417,466 filed on 19 October 2022.

### BACKGROUND

Tattoo application has been known for centuries. By placing dyes on the surface of the skin and then puncturing the skin with a needle, the ink or dyes are drawn into the wound created by the needle. The presence of ink or dyes in the wound stain the tissues making them adopt the color of the ink or dyes driven into the skin during the puncturing process. Once the wound is healed, the ink having been drawn into the dermal tissue, the extracellular matrix, and perhaps some cells, permanently stains the cells and surrounding tissue structures, causing the stained tissue to be visible from outside the dermis.

While prior tattooing processes were often simple procedures performed with crude instruments, as time has progressed, the single needle, hand application of tattoos has been augmented by mechanically and electrically driven oscillating or linear driving needle devices. These devices enable the tattoo artist or technician to vary the speed at which the needles drive the ink into the skin, as well as control the depth at which the needles penetrate the skin. Likewise, the inks and dyes have been improved allowing more skin safe and color variational products to be applied. Over time, as the technology has improved, in some cultures, large sections of the client's body have been tattooed, and the application and display of these tattoos has been celebrated and admired.

Prior application methods and environments increased both client and technician risk for infection and injury. As infection and transmission of diseases have been better understood, additional means of protecting the technician and client have been developed. These more sanitary methods have led to the creation of removable, single-use needle bundles that are disposed of after each use, preventing cross infection that was more prevalent in earlier single needle devices.

Presently, tattoo application involves practices that are increasingly artistic and implement relatively safe methods of body art application with many products on the market for application including mechanical devices for driving needles, sanitary inks and dyes, surgical type or other infection fighting methods to enhance the experience of the technician and the client alike. It is estimated that tattoos have been applied to 20% of the US population. However, many clients have desired to change their body art or have the art removed completely. This has resulted in a need for tattoo removal or tattoo alteration that requires the removal of the ink contained within the tissue structures on the client's body.

Several methods of tattoo removal have been developed over the years, beginning in the 1920's. Such prior methods determined that treatment sites for tattoo removal had to be spaced apart a specified distance so as to leave tissue bridges which allow each treatment site to be surrounded by active healthy tissue. These bridge areas between treatment sites allow blood born healing factors and cells to be supplied to the treatment site, thereby promoting the reconstruction of the tissue in the treatment site.

Early treatments were crude and often performed by hand using manual needle applications to create a wound on the skin of the client containing the tattoo ink thereby allowing the ink pigments to be released and to eventually be removed either with the blood in the wound or by being agglomerated into the eschar scab that formed as a result of the tissue damage caused by the needle-induced dermabrasion. If tissue bridges were not maintained during the removal process, the area being treated did not heal well and excessive scar tissue was formed during the healing processes.

Newer methods and options for treatment have improved the application and removal processes. However, there are drawbacks to these devices and methods. During tattoo application, planning and image placement using patterns and stencils require time and patience of both the technician and the client. While time spent during the treatment sessions is often mutually understood and accepted by both the technician and the client, the time spent planning the tattoo removal process on the other hand, is not as well accepted. Most clients, after making the decision to have a tattoo removed or altered are anxious to get the process started in order to move toward completion. Nevertheless, additional time is needed to ensure the correct approaches to the removal of darker pigments and stains and to establish that the skin bridges are properly healing without incurring detectable scarring.

Removal methods may require some planning to ensure correct placement of the isolated treatment sites, called tegulae, to maintain skin bridges and realize optimal healing. The use of a template or stencil as a planning tool, marking the treatment site is a major source of time spent during the tattoo removal process. There is a need for tools capable of assisting the technician to space the treatment sites without extensive prior planning, thus reducing the overall treatment time and ensuring that proper skin bridges are maintained between tegulae.

After planning is completed, the technician may use a superficial dermabrasion brush to abrade the tissue. The brushes may consist of bundled needles similar to those used in the tattoo application. The use of smaller needle bundles, typically about 1 to 2 mm in diameter, may require the technician to move the needle bundle in a circular motion to fully cover the planned treatment site, often a circular disk 5 to 6 mm in diameter. The technician physically moves the needle bundle laterally while the needle bundle oscillates axially in a circular spiral pattern to fully cover each tegula.

The process for removal may require the skin of the client to be stretched so that the oscillating needles more easily and consistently puncture the skin. Penetration depth of the needles may be controlled by keeping the end of a needle cartridge aperture in contact with the working dermal surface or exterior skin surface. Once the abrasion and tissue disruption from the needles has begun, the working dermal surface deepens due to disruption of the tissue structure. Once the treatment area is fully and evenly disrupted, the loose tissue and exudate from the wound is wiped away and the treatment proceeds to the next tegula.

The circular motion and velocity of the strokes are subjective techniques that vary from technician to technician, often influencing the success of the ink removal process. Moving too slow may increase the wound disruption and the likelihood of scarring while moving too fast may not disrupt the tissue enough to stimulate complete ink removal. There may also be disadvantages in using a larger needle bundle with known devices and methods. In particular, the coverage required for even abrasion may not be achieved since needles are unable to be placed side to side in tight arrangements without spacing due to manufacturing constraints and the difficulty in holding needles in such arrangements during assembly. Retention may become a problem since there is a difficulty in securing needles to one another and to a mechanism that can oscillate them in high-speed fashion needed for good penetration and abrasion.

Accordingly, there is a need for a consistent method of treatment that incorporates axial oscillation along with the lateral motion of the needles that is objectively administered and that can treat singular repeatable wound areas thereby making the treatment process faster and more consistent. Specifically, a device is needed with the capability to treat both large and small treatment areas, without having to spend inordinate amounts of time stenciling, marking, and planning the treatment. Moreover, a device is needed that can treat these areas with a consistent treatment diameter, motion, and abrasion signature thereby ensuring consistent results in both superficial dermabrasion and dermal needling. The device and method of use disclosed herein solves these problems and others.
US 2021/0386987 A1 relates to robotic tattooing systems and related technologies. CN 101,897,602 B relates to an automatic hair tattooing method, a hair tattooing machine produced by same, and application of the same. US 2021/0244928 A1 relates to a pen style wireless tattoo machine, system, and kits. US 2006/0041241 A1 relates to a drill device and method for forming microconduits. US 9,055,158 B2 relates to eradication of pigmentation and scar tissue.

### SUMMARY

The aforementioned objectives are achieved according to the invention by means of a system for use in the application and removal of tattoos and other skin treatments according to claim 1. Preferred embodiments are defined in the dependent claims.

Embodiments of the system may automate treatment procedures by programming or controlling one or more of four independent degrees of freedom: three mechanical brush motions and a facilitating fluid flow. An advantage of the system is that it may make treatment faster and simpler for the technician and less dependent on subjective technician skill. Because the various drive motors and the facilitating fluid pump are controlled independently, the system can be programmed for different applications and procedures: for example, from superficial dermabrasion for tattoo removal and scar revision to microneedling for skin tightening and oscillatory needling for tattoo application.

Embodiments of the system may comprise a control element, a drive element, a handpiece element, a cartridge element, and a fluid element.

The control element may be a fully programmable controller comprising an electrically powered digital or microprocessor for controlling the various parts of the system. The control element may be positioned with the drive motors and fluid pump in a common housing or located remotely and connected either by wire or Bluetooth. The control element may comprise a graphic display, with touchscreen or selection buttons that can be activated by a gloved hand, for choosing a procedure, viewing and/or overriding procedure parameters (depth, frequency, etc.), and viewing procedure results (cumulative treatment time, number of tegulae treated, pumped fluid volume used, etc.). In some embodiments, the control element is a wrist controller (connected by Bluetooth and worn by the technician) for customized functionality. All wrist controller functions are also available from the controller element and graphic display.

The control element may comprise a foot controller or another sensor used to initiate the selected treatment program. The initiation sensor or switch may be located on the handpiece, or the initiation sensor may be integrated into the wrist controller, which could sense wrist tendon movement, tension, or muscle electrical activity. The controller may have several treatment protocols and type parameters programmed within. The controller may be capable or accepting new and different software programs that may not have been originally contemplated. The presentation of these different treatment options shown on a graphic display allow the user to select a needling procedure, tattoo application, tattoo removal etc., by activating the touch screen. Once selected the parameters within this device will be translated to the drive system and once the correct cartridge and is attached, the technician may perform the selected treatment. During treatment, the technician can vary some of the selected parameters such as oscillation speed, rotational angle of the needle bundle and the depth of needle penetration by using the wrist controller or also by using the graphic display on the controller. The controller may control several functions of the drive and pump at once and blend them to a pre-programmed treatment or may only use one of the drive functions at a time. Velocity and speed of needle rotation would be controlled and could be preset functions or programmable by the technician if such actions were unlocked to allow such access.

The drive element may comprise an oscillation drive, a distance (or depth) drive, a rotation drive, and a flexible connecting shaft. First, the oscillation drive produces the axial oscillatory motion. It sets the stroke (total peak- to-peak travel) and controls the oscillatory penetration frequency of the needles. Second, the distance (or depth) drive sets the extension of the oscillatory motion or the distance of the needle extension from the handpiece, thereby affecting the maximum needle penetration depth for oscillatory motion. Since the speed of this drive is variable, it also drives non-oscillating needles through slower microneedling penetrations and extractions. Third, the rotation drive rotates the needle bundle during the treatment process and can be controlled to make complete or predetermined angular rotations or to set angular position of the needle bundle, as for example, when used for tattoo application. Fourth, the flexible drive shaft mechanically transmits the three independent motions (oscillation, depth, rotation) to the handpiece element.

Depending upon the embodiment, the independent motions in the flexible mechanical transmission may be combined in one mechanical element: one flexible tube or solid shaft that simultaneously transmits rotations and axial motions (oscillatory and distance). Other embodiments can have the motion split and transmitted between two mechanical elements: an outer torsional tube to transmit rotation, and an inner shaft to transmit axial motions (oscillatory and distance). Flexible mechanical transmission motions can also be split between one mechanical and one hydraulic element: an outer torsional tube to transmit rotation, and an inner hydraulic column to transmit axial motions (oscillatory and distance).

The handpiece element may be couplable to the flexible drive shaft and accept a disposable needle cartridge. The handpiece may smoothly transmit the axial and rotational motions (generated by the system and transmitted by the flexible drive shaft) into the cartridge needle bundle creating both axial and rotation motions of the needle bundle.

At the distal end of the handpiece is an oriented mating receptacle for the needle cartridge, which cartridge may be locked in place (and later removed) with a quarter-turn twist or other disconnect motion. The mating receptacle may be oriented so that when locked in place the fluid port on the cartridge consistently places the feed tube in a convenient position, as for example between the technician's thumb and fingers gripping the handpiece and cartridge. The mating receptacle may be embodied as an oriented obround or other shape.

The handpiece may contain an axial position encoder to programmatically compensate for unpredictable axial motions caused by arbitrary reorientations of the handpiece or the flexible connecting shaft. The encoder may measure the instantaneous needle position, or just the needle position at the peaks of the oscillatory extension.

The axial position encoder may be zeroed each time a cartridge is inserted. Zero is defined when the maximum extension of needle tips (while oscillating) lies just below the plane of the cartridge tube tip. An optical or electrical device (for example, a conducting mesh screen) can be used to make this zero detection, which is made with the needles oscillating, typically before facilitating fluid enters the cartridge.

Whenever de-energized, the oscillatory drive may come to rest at the full-retraction position and may be locked in place. This prevents technician needle-stick injuries, allows repositioning of the cartridge without lifting it from the dermal surface, and prevents axial slippage when microneedling with the depth drive.

The cartridge element may comprise a housing; a multifaceted shaft (polygonal, splined, etc.) keyed to the handpiece drive shaft and connected to a needle platen; a spring; a platen that holds and configures the needles, several sharpened and polished, stainless steel, solid needles; and a dermal scrapper and support that rotates with the platen but is fixed axially. The housing, shaft, platen, and scrapper/support may all be injection molded.

The cartridge element may be a disposable plastic molded assembly with one or two movable parts in the front (distal end) center, that contains a needle bundle. Needles may be solid sharpened and polished stainless steel medical grade needles, not hollow in that no fluids is allowed to traverse through them. They may be attached to a movable platen or plunger that is allowed to move in an axial and rotational fashion in line with the centerline of the cartridge. The second movable element may only rotate and may be used to scrape away abraded debris and constrain the working dermal surface to the tube-tip plane.

In embodiments of the cartridge, the housing cylindrical with a protrusion on the top side that has a passage to allow fluids to travel to the distal end of the cartridge, and finger grips on the sides. The platen is retained within the housing and has limited travel in the axial direction. There is a spring that helps keep the needle bundle and platen assembly in proper orientation and/or recessed within the cartridge. There is a small hexagonal or other shaped shaft extending from the rear of the cartridge that when pressed or rotated, allows the needle bundle to move linearly or axially as the forces impinged cause its movement. The outside rear (proximal end) of the cartridge comprises an obround or ramp obround feature that allows the cartridge to be inserted into the distal end of the handpiece. The obround ramp feature permits the cartridge to be rotated into the end of the handpiece and be retained by interference of the ramp and obround features. The ramp may be shaped to another form of receptacle that mates both cartridge and handpiece together and forces the cartridge to maintain orientation during use. This receptacle would allow other sized or functional cartridges to be so inserted and retained and to maintain a similar orientation.

The proximal end of the cartridge housing may lock into the oriented mating receptacle, while its distal end (where the needles extend) may accept a transparent layout disc. The housing top side may also feature a fluid port that communicates with the needle compartment. The fluid port connects and seals to a feed tube that transports the facilitating liquid solution into the needle compartment. Because technicians tend to grip the device as low as possible-even the cartridge itself-finger grips may be molded into both sides of the cartridge housing.

In further embodiments of the cartridge, the distal end of the cartridge is shaped to allow a part to be attached. Layout of properly spaced tegula, without using a template or marking skin in preparation for treatment, can be accomplished with a layout disc, which is attached (and later removed if desired) to the distal end of the needle cartridge. This part may be transparent and may comprises a hat-shaped portion with a flat circular brim and a hole in the middle to allow the part to be attached to the cartridge housing. When attached, the brim is flush with the distal end of the needle cartridge. Deformable plastic elements may be used to keep the hat attached and still allow its removal. The transparent hat may be used as a tegular layout aid, which use is described below.

Embodiments of the hat shaped layout disc or spacer ring may allow the technician to space the treatment sites away from one another to ensure proper skin bridges to facilitate the healing process. The use of such a part allows the technician to space the treatment sites without having to mark the skin of the client in preparation for the treatment. This may be accomplished by repositioning the handpiece and cartridge so the disk brim does not overlap any portion of skin already treated. This may provide an advantage by saving time for the treatment cycle, and considering the treatments might be charged hourly, it will also save the client money and time.

A restricting orifice or passage can be molded into the cartridge housing at the end of the fluid path to restrict the flow of the facilitating liquid solution so it will not leak out of the fluid path when unpressurized. The restricting orifice may enable exacting momentary control of fluid flow via the programmed positive displacement pump. To conserve fluid and to prevent it from overflowing tegulae (which is an advantage because the facilitating liquid solution is often a dermal irritant), a volumetric measure of the liquid could be squirted into the needle compartment at the beginning of each tegula cutting operation.

A platen and needle bundle may reside in or be affixed to a front or distal end of the cartridge. The needle bundle can be configured to suit a particular use or procedure. Needle patterns can be configured as single or multiple lines, crosses, arcs, circles, and combinations of these, or in any other way that best suits its intended use. Needles in the pattern can be spaced differently to compensate for nonuniformity in coverage with rotation. Needles can vary in length and diameter to provide the required flexibility and reach. Graduated or stepped flexibility can be produced with graduated or stepped needle diameters. For example, significant needle spacing combined with purely axial penetration (without rotation or oscillation) allows for penetration with minimal damage to the skin to be used as an aesthetic microneedling treatment to remove wrinkles or tighten the skin. More closely spaced needles that are oscillated and rotated simultaneously can be used for tattoo removal and other superficial dermabrasion procedures.

Compact shapes and patterns of needles can be used for tattoo application. Needle patterns that are not rotationally symmetric (like single lines or magnum double lines) can be rotated to particular azimuthal angles to suit the inked line or swath being laid down. Many types of treatments that can be performed with embodiments of this device. In addition to the needle bundle being sized and spaced differently, the diameter of the needle bundle may be altered to be up to or greater than 6 mm and down to or less than 1 mm in diameter. Additionally, needles can be increased in length or decreased in diameter for more flexibility.

The needle cartridge may contain an element that rotates with the platen and needle bundle but does not move axially. The rotating element may press against the dermal working surface to scrape away abraded tissue and to keep the working surface from moving axially during the tegula cutting operation. The tissue touching distal plane of the rotating element lies on or close to the distal plane of the cartridge tube tip.

Embodiments of the cartridge may dispense a facilitating fluid, such as Teprsol ^{®}, or even ink when tattooing or placing permanent makeup. If the treatment requires small amounts of the fluid such as ink, the fluid can be contained in the small reservoir that attaches directly to or close to the cartridge. The other end of the cartridge is attached via the feed tube to the positive-displacement pump that drives a hydraulic fluid, such as water, which in turn drives the facilitating fluid or ink. If the hydraulic and facilitating fluids are miscible or may not touch for any other reason, the small reservoir may consist of the flexible bag containing the facilitating fluid encased by a rigid container accepting the hydraulic fluid.

Surface tension may keep a water-based liquid from flowing out of the cartridge tube tip when unpressurized for cartridges with small and compact needle bundles. For embodiments comprising larger needle bundles where unpressurized liquid may leak out of the cartridge tip, control may be accomplished by programming a pump to squirt a volume of liquid briefly at the beginning of the programmed tegula cutting operation. To retain liquid in the fluid path and allow momentary squirting, a restrictive orifice or passage can be molded into the cartridge at the end of the fluid path.

The fluid element, which is responsible for feeding the needle cartridge with a programmed flow of the facilitating liquid solution (Teprsol ^{®}, for example), may comprise a fluid container, a fluid pump, and a feed tube. The fluid pump may be a positive displacement pump, and the feed tube may comprise a tube or tubing set and may connect the fluid container with the needle cartridge. Embodiments may comprise a metered positive-displacement pump that can dispense facilitating fluids to the cartridge element using the feed tube and Luer lock seals or other seals where needed, including on the cartridge. An advantage of such embodiments is that the liquid volume flow rate is controllable independent of the flow pressure required. When the fluid pump is integrated into and controlled by the system, liquid flow can be restricted to tegula-cutting operations, thereby saving fluid and preventing spills, tegular overflows, or dripping normally associated with these treatments.

The sterile fluid path, comprising all system components that touch the facilitating liquid solution which in turn flows onto treated skin, may comprise three parts already mentioned: the fluid container, the feed tube, and the needle cartridge. All may be sterilizable, single-use, disposable consumables.

The feed tube connection between the fluid container and needle cartridge can be implemented in several ways. For example, the feed tube could be a commercial tubing set connected by Luer locks on both ends. Alternatively, it may be initially manufactured together and packaged with the sterile cartridge. Furthermore, it may connect to a pigtail which is part of the needle cartridge.

The fluid pump can be any positive displacement pump that preserves a sterile fluid path. Examples include syringe pumps driving liquid-filled syringes and peristaltic (tube and pinch-roller) pumps. Positive displacement pumps provide a constant (or programmable) volume flow rate independent of the pressure required to drive the fluid.

Some embodiments of the system may comprise: 1) a control element, 2) a drive element, 3) a handpiece element and 4) a cartridge element. A fifth, fluid element, may be physically combined with the system or it may stand physically and functionally independent of the system.

Some embodiments of the system may comprise a handpiece, a controller, a power section, and a disposable treatment cartridge. The treatment possibilities are not limited to tattoo removal, but with the variable control functions this device will be able to perform, additional areas of use are contemplated such as tattoo removal, micro-needling, tissue abrasive uses, skin tightening, wrinkle removal, and other cosmetic treatments.

Turning to functionality, the controller monitors and adjusts the various functions, memory, data communication, visual representation, outcome prediction, and fluid disbursement during the treatment. The controller may be a self-contained unit or comprise a tablet that functions as the controller. Embodiments comprising a tablet feature increased portability around the treatment site and may be used for image capture of treatment sites on the patient. The functions of the system in all embodiments may be established and monitored by the controller. The controller may also comprise a small handheld or wearable interface configured to communicate with the controller. The controller may further comprise a graphic display, a user interface, a processing/control section, a power supply section, a fluid pump section, and a microprocessor.

The microprocessor may be configured to control and coordinate all system functions and communicate between various parts of the device during operation. The microprocessor will interface with the graphic controller in providing information via the graphic display to the user, both in graphic format and audio communications. The selections made on the graphic interface for operation will be communicated to the microprocessor for control of the various parts of the system. The microprocessor will control the library of operations and functions contained within the memory to allow the user access to images, programs, history, or other features that enable ease of operation. Should a user desire to use programs in memory for treatment options, such items will be retrieved by the microprocessor and communicated to the graphic interface for display and interaction by the user. if the user decides to create programs for operation of the device, such interface can be made in the graphical display, but will be evaluated prior to operation to ensure the program being created will be achievable within hardware parameters and be safe for operation, with a warning to the operator if the program has not been tested, is approved or clinically viable.

The microprocessor may be configured to process, store, retrieve, and download images and patient history from other systems wherein Health Insurance Portability and Accountability Act ("HIPPA") complianty protections are enacted. The microprocessor may handle communications between sections of the device via a direct cable link, as with the pump section, through the multi-use handpiece, or via Bluetooth and other wireless communication protocols in embodiments featuring the wearable controller or the remote graphic tablet. The microprocessor may monitor all operations of the sections during setup and treatment to ensure safe operation and to ensure that the various functions under control are operating with the parameters established by the technician, stored requirements, and incoming feedback from various sensors on the device. Accordingly, the microprocessor ensures that device operations are consistent with clinical requirements and conducted safely and to ensure protection to the patient and user during operation.

The control system may be contained within the handpiece and be in communication during setup and operation. The controls and processing within the handpiece may communicate directly with the processor via a hard wire connection using communications methods such as Controller Area Network (CAN bus) or other equivalent methods. This way all operation of the handpiece is known and monitored for safe operation, and it also provides a buffer against loss of control due to communication interference with systems such as Bluetooth or wi-fi. In addition to monitoring handpiece functions, the microprocessor may control and monitor the fluid pump functions. This puts the pump in direct control to allow coordinated operation of the two during treatment.

Some embodiments of the disclosure may comprise a graphical user interface housed in a separate tablet or within the main housing itself, configured to provide information to the user regarding chosen parameters, operating conditions, and feedback for safely operating the system. The graphic display may be part of a base system or be enclosed within a tablet enclosure. Being in the tablet enclosure enables the tablet to be moved to a treatment site, positioned near the technician, or used for remote reasons such as for image capture, gathering patient input data prior to treatments beginning or to be able to review patient data in treatment preparation. The graphic aspect of the device may include a hard keypad or keyboard to input data, manipulate files and display or retrieve files.

The graphic display may contain a graphic processor to communicate with the microprocessor and to display information from the microprocessor, such as files, parameters, signals or other information both in graphic format or audible formats.

The graphic display may convert information from the graphic processor into visual information for use by the technician, including for: displaying graphically the treatment parameters that are set for an upcoming treatment, making modifications to a treatment setting and locating and displaying treatment settings, and displaying information for safe operation of the device. Since the technician may be busy or distracted performing treatments, the graphic display can provide audible and graphic warnings to the technician about the system operation and to report any malfunctions or other deviations for the settings from established parameters. Other items that can be displayed include time tracking, suggestions for treatment options, patient history, preferred/effective treatments, and controls to adjust treatment parameters to tailor the treatment to the patient skin type. As other treatment options become apparent for use with this device, the graphic interface may show imagery of what is assembled, procedure directions, and options for upcoming treatments.

Some embodiments of the disclosure may comprise a power input and control. The power input section of the device may be capable voltage input ranging from 100 thru 250 VAC. The section can have a filter and conversion section that actively converts the AC voltages to lower and/or DC voltages that can be used in the output sections. The power output section of the device may receive the lower AC or DC voltages from the input section and convert those voltages for use in the control section of the system, the handpiece, and the pump. If the control system incorporates a removable tablet or wearable wrist controller, the output section may contain charging circuits to ensure the electrical charge within the batteries in those items is maintained at proper voltages for operation.

Embodiments of the disclosure may comprise a wearable wrist controller. Alternatively, the controller may also have a wrist worn part that may be linked to the controller and communicate operational status of the system. The wrist controller may have a graphical screen to display images of the system status and other operational settings and characteristics. The wrist controller may be linked by Bluetooth or by other communications protocol to the main controller. Embodiments of the battery in the wrist controller can be recharged by placing the wrist controller in a designated location containing a charging interface or connection on the main controller or attached to a charging wire or cable when not in use. The wrist controller may be cleanable, sterilizable, and sealed to prevent ingress of fluids. The screen may comprise virtual buttons that are touch activated through a thin glove allowing the user to wear hand protection when using the device, provided that the glove allows the user to see the application running.

Embodiments of the disclosure may comprise an integrated fluidic pump for fluid flow. The device in the controller may include a fluidic metering pump that can dispense Teprsol^{®} or other fluids to the cartridge element using the fluid tubing set and aperture of the cartridge. An advantage to using this type of pump with the system, is that the fluid flow, will be controllable by the clinician/artist. Additionally, the flow of the pump may be controlled to stop when the treatment is paused or concluded, thereby saving fluid and preventing spills or dripping that can be associated with these treatments. To prevent the treatment and needling without fluid present, the flow of the pump may be started prior to oscillation motion of the needles.

Embodiments of the disclosure may comprise a multi-use handpiece. The multi-use handpiece/drive system may comprise three distinct parts each independently controlled and operated by an internal controller: 1) an oscillation drive that produces the oscillation motion, or the forward and back motion, of the needle bundle, in essence the in and out motion of needles when impinging with the skin; 2) a distance drive, varying stroke extension of the oscillations or the distance the needle bundle travels which translates into the depth of penetration of the needles in the recipient's dermis; 3) the rotational drive which translates into the rotation of the needle bundle around the bundle and handpiece central axis during the treatment.

Each of the drive components may be controlled via an electrically powered digital controller and monitored for proper function and operation. While the drive components will often act in tandem, each individual drive component within the handpiece may operate independently for certain treatment parameters. The multi-use handpiece system may be connected to the power supply and controller via a cable that provides the power and signals for controlling the handpiece functions.

The drive components may comprise two stepper-controlled motors and a DC brushless or brushed motor, including: 1) a rotational drive comprising a first stepper-controlled motor configured to control a rotation direction speed and angle of the needle bundle in the cartridge; 2) a distance drive comprising a second stepper-controlled motor configured to control a distance setting or position of the oscillation motor setting the distance of the needle bundle from the end of the cartridge; and 3) an oscillation drive comprising a DC brushless or brushed motor controlling the oscillation of the needle bundle in the cartridge.

The oscillation motor may be a DC motor that can operate at varying speeds of rotation, either by motor control or pulse width modulation of the signal, or by simply varying the voltage of the motor. The speed of rotation of this motor may be selectable by the clinician during the treatment cycle. The oscillation motor drives an eccentric crank that drives a linear shaft, which motion ultimately impinges on the end of the cartridge thereby causing the cartridge needle bundle within the cartridge to move either distally or proximally. This oscillation speed assists the disruption of the dermis cells containing the tattoo ink. Embodiments of the system may preferably operate at between 4,000 and 14,000 oscillations per minute and more preferably at about 10,000 oscillations per minute, though the system may operate above or below the preferred range. If the operator is performing a needling treatment that does not require that frequency of oscillations, the speed of this motor may be reduced to accommodate the type of treatment being performed.

The control of this oscillating motor can be varied and controlled by manually controlling the control panel settings or by using the remote controller or wrist worn controller. In such motors, speed and control may be maintained by using feedback within the motor housing with hall effect sensors or other methods allowing the motor speed and armature position to be known and maintained during operation. In this device, knowing the position of the armature may ensure that the needles will be fully retracted and are not in the extended position when the operator positions the device during treatment. Also, this motor may allow for very slow speed operation and control, to allow the armature position to be used for cartridge installation and connection, and for cartridge disconnection following treatment in preparation for disposal.

Alternatively, the axial drive and connecting shaft assembly may comprise hydraulic coupling. In such embodiments, the oscillation motor drives a hydraulic transmitting piston that oscillates a liquid within the flexible connecting tube (which tube also torsionally transmits the required rotation), and the motion of the liquid transfers to the handpiece, it also being set up to contain the liquid and a receiving piston. The hydraulic oscillation activates the cartridge needle bundle in axial motion the same as if the oscillation motor were operating the flexible rod in the connection shaft.

The rotation of the needle bundle mounted within the cartridge may be controlled by a second motor, such as a stepping motor. When this stepper motor shaft rotates, a needle bundle located within the cartridge is rotated. This motor is connected to the connecting shaft with a belt drive or gear drive convenient to the design of the system. When axial and rotational motion transmission is split, this motor is connected to an outer shank on the connecting shaft. When the motor shaft rotates, the outer shank within the connecting shaft rotates. The rotational motion is translated down the connecting shaft into the handpiece and eventually to the needle bundle located within the cartridge. The speed and rotational angle of this motor may be controlled by the controller element and its specific angles and speed may be part of the programming for specific treatment parameters programmed within the control element. Another embodiment of motion control and transmission combines the axial and rotational motion prior to entering the connecting shaft into a central internal flexible shaft housed within the sheath. This simplification allows a smaller diameter, more flexible connecting shaft while retaining the axial and rotational motions generated.

By controlling both motors simultaneously, a compound motion of the needle bundle may be obtained which can be suitable for many treatment parameters besides only tattoo removal or application. In prior systems, the clinician may be required to move the small needle bundle in a circular motion to cause abrasion of the dermis in the treatment site. The rotational action of the present system may mimic the circular motion that is performed by the clinician during prior tattoo removal treatments. An advantage of this rotational motion control and linear motion control is that there can be more consistency from treatment site to treatment site, making the healing and dermis abrasion more consistent. Other advantages might be that less time is used by the clinician during the treatment cycle by ensuring all the treatment parameters were met equally.

Advantages of the compound motion may include different tissue effects as the treatment progresses or may allow the technician to match treatment parameters based upon the patient's skin toughness, or flexibility to better suit the treatment procedure. This may also save significant time in treatment, since the operator does not have to move the smaller needle bundle in circular motions as with alternative devices. The flow of a facilitating fluid, such as Teprsol ^{®}, may be determined by the positive-displacement pump. When driven by the system controller, the pump provides timed flow control for each tegula-cutting operation; fluid is squirted into the needle chamber only at the beginning of each operation. Alternately, the pump can be a stand-alone device that produces a constant volume flow rate during the entire treatment. With the introduction of the fluid at the point of treatment, the same tissue effects and ablation may be obtained.

The use of the third motor located within the drive element may vary the distance of the oscillation motor either closer to the cartridge tip or away from the cartridge tip. The distance element controlled by this motor may change the distance of the oscillation motor from the tip of the cartridge, thereby changing the depth of penetration of the needles as they oscillate. It may be desirable to have a range of adjustment that may be 0 to 3mm depth control. 1.5 mm may preferably be used for tattoo removal. The penetration of the needles may be considerably less for other micro-needle treatments such as used for wrinkle removal or skin tightening.

This third stepper motor may drive a carriage that moves the oscillation motor toward or away from the distal end of the cartridge tip. By moving the position of this motor, the position of the needle bundle in the cartridge is changed also, changing depth of needle penetration either reducing it or increasing it. The distance may be read by an external optical encoder or can be done by the stepper provided it has an encoder attached. The distance can be changed during operation but may preferably be set at the beginning of the treatment. The depth control may be located on the controller panel or the remote panel.

The motors may be programmed to act independently as well as collectively. Certain treatments of needling may be programmed to operate only using the distance motor since speed of oscillation would be too high and cause too much tissue and needle interaction. The slow in and out control of the oscillation motor would allow the needle penetration to be slower and more controlled for example as used in a micro-needling procedure. To avoid depth slippage, the oscillation motor would be held fixed (preferably at is full-retraction position).

In addition to these functions, the oscillation drive system may include a method of "zeroing" the needle bundle after treatment or before treatment, to allow the calibration and true measurement of the distance the needles by be set to travel from the distal end of the device. This zeroing may ensure that what the clinician sets for needle penetration would be accurately performed by the device. Such measuring capability may be included in the drive section of the device or a handpiece rest, in that the cartridge tip could be temporarily inserted into the measuring device for the initial zeroing measurement. Alternatively, such measuring capability may be be included in the control section of the device.

As discussed previously, the handpiece control may include a method for needle retraction when the treatment is paused or concluded. This would allow the needle bundle to be positioned on the recipient's skin surface and allow minor movements of the clinician without needle penetration, prior to beginning the treatments or for restarting the treatments in another location. This could be embodied as a spring drive or mechanical positioner for the oscillation drive motor to ensure the cam and drive are correctly positioned in a retracted position in between or upon completion of a treatment. When de-energized, the oscillation motor should be locked so that depth slippage is eliminated. As discussed, information regarding the armature position within in the brushless motor may allow for positioning the needle bundle in the retracted position for the oscillation drive motor will ensure the crank and drive are correctly positioned in a retracted position in between or upon completion of a treatment.

Some embodiments of the disclosure may comprise a disposable treatment cartridge. The disposable treatment cartridge may be a single use plastic multi-part assembly that is attached to the handpiece for performing a tattoo removal procedure or other dermatological treatment depending upon the assembly configuration.

This disposable treatment cartridge, when installed on the handpiece, may be used to treat, abrade, needle, or puncture human tissue at a specific site or area when the proximal end of the device is placed in contact with the patient tissue and the handpiece drive is activated. In tattoo removal, the treatment site is the result of abrasion of tissue from the epidermis is referred to a tegulae. The disposable treatment cartridge may comprise blades or needles or a combination thereof that are driven against the epidermis. The materials used in the construction of the disposable treatment cartridge may comprise medical grade thermoplastics and surgical stainless-steel components. The motion and impingement of the blades or needles in contact with the epidermis abrades the tissue in contact and causes the abrasion wound or tegula. The size of the treatment area produced by this device may correspond to the diameter of the bore and abrasion producing items within the disposable treatment cartridge.

The disposable treatment cartridge as described herein may comprise an assembly of several injection molded parts, a compression spring and a needle arrangement or a blade. The blade will be discussed in further detail herein. The cartridge may comprise an exterior housing that has a central bore and an oblique bore that is angled and joins the central bore near the treatment aperture of the cartridge. Through this aperture, fluid can be introduced into the treatment site, and may be pumped from a sterile source, through a tubing set, and into this bore. A narrow aperture prior to joining the central bore may small enough to prevent fluid dripping and flowing when not being pumped. This fluid application may be used in the tattoo removal process, in that it helps encourage the creation of clean abrasive wounds that scab over cleanly and help the healing process to occur. Even though tattoo removal applications may require the fluid application, other dermatological treatments may not require fluid dispensing, and as such a fluid port might not be required on all embodiments of this device.

Needle arrangements may comprise closely packed arrangements, open spacing of needles within the cartridge, lines, rows of needles, or varying lengths of needles resulting on some needles penetrating more than others during oscillation. Such arrangements for large, spaced needles may be used for active needling treatments to spur the development of collogen in facial tissues.

Other embodiments of the abrasion portions of the cartridge for tattoo removal may comprise a thin surgical blade or combination of blades that protrude from the needle driving surface that can impinge the tissue of the patient. Such blades may be tapered on the tip in one direction while having a greater width in another direction such as with a razor blade. Other versions may be laser cut or etched to have the face of the blade cut through with a needle profile resulting in a sharp, sawtooth pattern of needle forms. Such blades may have the sharpened tips of the blades vary in spacing. The spacing may form points similar to a series of side by side needles or may comprise other needle spacing ratios, such as one needle per for each needle width or one needle for two needle widths and so forth. The spacing may also be mathematically determined to be logarithmically spaced that may result in even abrasion when oscillation and rotational motions are applied. Embodiments of the blade may be formed with needle tips of equal length. Alternatively, the blade may be cut such that some of the needles are longer than others, or such that a curved or angled pattern form along an axis of the tips of the needles. Other variations may include deep cuts between the needle forms of the blade to increase flexing and bending.

Some embodiments of the blade application on the needle holder surface may be a grouping of blades in an arrangement such as radially spacing, even rows of blades or staggered blade rows that when oscillated and rotated simultaneously cause an even abrasion with consistent depth and tissue removal. The variations of such a blade arrangement have may possible arrangements, shapes, and options that can be used to fit other applications for other abrasive or needling applications.

The construction of the disposable device may comprise a main housing, drive rod, blade holder, and interface driver. In some embodiments, a spring may be used to ensure the assembly internal components are maintained in a proper position and returned to a resting position or safe position upon removal from the handpiece.

The disposable device may comprise a single piece housing that has a central bore wherein needles, blades or other items used for abrasion are placed. The outside of the housing may comprise features that allow the disposable device to be installed and held in place within a mating aperture in the handpiece. Features for correct orientation and retention comprising of molded protrusions and holes or other features may aid in installing the disposable device in a specific orientation within the handpiece and locking that position during use. These features reduce incorrect installation and subsequent confusion that may arise of incorrect use. This outer molded housing also has an oblique protrusion that incorporates an angled tubular bore that joins to the central bore of the housing near the proximal aperture of the disposable.

**In** embodiments of the system, a driving rod may be positioned within a central bore. The driving rod interfaces with a motor driven pushrod located in the handpiece. The pushrod within the handpiece pushes on the driving rod. The design of the pushrod in the handpiece also transmits rotational motion to the drive rod within the disposable during a treatment cycle. The design of the drive rod and freedom of motion it has within the molded housing allow the drive rod to be moved in rotation while also oscillating linearly. The combination of these features allows for the desired abrasion to occur at the treatment site. In addition, since these motions are independently controllable within the handpiece, the variety of treatment options, as mentioned previously are available based upon the needle or blade configuration installed on the end of the drive rod.

The drive rod and internal apertures of the disposable may preferably be 5mm for tattoo removal, but larger diameter bores, and drive rods can be used allow a more varied treatment option depending upon the needs of the patient. The distal end of the housing may comprise an integral spring that places the cartridge housing in tension and helps maintain the integrity of the cartridge during use and helps in ejection during removal from the handpiece. Also on the proximal end is an annular disk feature that extends outward normal to the axis of the cartridge. this disk diameter is calculated to ensure that the technician positions the cartridge tor each subsequent tegula to be spaced away from previous tegulae to ensure that healing will occur. The cartridge assembly may also comprise within its design a locking tab feature that restricts drive rod motion, thereby preventing accidental cuts or needle sticks to the technician during installation. Once installed the locking tab is removed and treatment can occur.

Returning to the connecting shaft, the shaft may comprise a flexible conduit comprising two elements that translate motion from the drive elements to the handpiece and eventually to the needle bundle located within the cartridge. The ends of the connecting shaft may have a screw or other similar type of attachment method that allows the connecting shaft to be removed or separated from the other components for service, replacement or for upgrade. Embodiments of the shaft comprises an outer sheath that may be polymer coated for maintenance and may contain a spiral flat wire support within the polymer coating. A further layer may comprise a flexible tube that is polymer material, or alternatively, a spiral wire flexible tube that can rotate within the outer covering. Each end of the tubing may comprise a mating feature, either male or female, that can mate with a corresponding feature, one in the drive element that would be attached to the rotational drive motor in the drive section and a similar type of feature in the proximal attachment end of the handpiece. When driven by the rotational motor in the drive section, the rotational motion will be translated into rotational motion in the handpiece.

In further embodiments, within the center of the rotation portion of the connecting shaft, which is tubular and hollow, a flexible metal rod may be located. This rod may move in a linear motion within the hollow rotational element within the shaft assembly. One end of this rod will be attached to the oscillation drive motor and the other end will drive a mating element located within the handpiece. This part within the handpiece will be free to move linearly and be free to move rotationally also. Being able to do both will allow interface with the cartridge and drive the needle bundle both linearly and rotationally at the same time or to drive them individually as desired. In further embodiments, the flexible metal rod may be constrained for both axial motion and rotation at the drive end, allowing the combined motions, both axially and rotationally to be combined on the drive end, thereby simplifying the connection shaft design and components.

In additional embodiments, the handpiece may be located at the distal end of the connecting shaft. The proximal end of the handpiece may comprise a threaded or quick connect receptacle to interface with the connecting shaft. This threaded or quick connect feature allows the handpiece to be separated from the system for disinfecting, cleaning, or sterilization. It also allows a handpiece of a different type to be applied should a special treatment requirement be required by the client needs. The handpiece contains a gender specific mating part that interfaces with the connecting shaft assembly that translates rotational motion from the connecting shaft to the proximal end of the cartridge. This part, after the area where the motions are sequenced within the handpiece, allows the cartridge needle bundle to be driven in rotational or axial motions as determined by the control and drive elements of the system.

The handpiece may comprise an external grip surface that allows the user some comfort by absorbing vibrations encountered during the treatment process. This surface may be tactile or more rigid. A programmed treatment initiation switch may be integrated into the handpiece.

The distal end of the handpiece may comprise an opening that is obround on the axis of the handpiece. This obround feature on the handpiece allows a cartridge with a similar obround feature to be rotated causing the obround features to interlock and remain rigidly held. An advantage of the obround feature is to allow the locking of a cartridge into the handpiece in a simple twisting motion. Removal of the cartridge would be the reverse of this twist, thereby freeing the cartridge from the handpiece. The angle of the obround relative to the planes and axes of the handpiece allows the cartridge to be locked in similar, predictable positions for use by the operator. This positions the cartridge to make sure the fluid delivery is located between the thumb and index finger of the user. The handpiece can be used by right or left-handed users as there are no features on the handpiece that are located on one side only.

Another version of the handpiece may incorporate a distance determining mechanism to control the distance and range of impingement of the linear drive rod on the back of the handpiece thereby setting the distance of linear travel of the drive features in the handpiece. The effect of this setting would be a control on the depth of penetration of the needle bundle as it impinges on the skin of the client. The handpiece may comprise markings indicating an established measurement related to rotations of the adjustment feature vs distance. This distance determining mechanism may comprise a thread. The materials of the handpiece may be metallic or plastic depending upon the desired manufacturing methods and cost of the device.

Since the cartridge may be a molded plastic assembly, it may be low cost and designated for single use only. Advantages of such embodiments are that there is less potential for cross infection from client to client and the cost savings for not having to disinfect the device from treatment to treatment. The technician would be able to have a selection of these cartridges and be able to choose the cartridge tailored for the treatment desired. The proper cartridge may be depicted on the graphical display on the control element thereby reducing the potential errors for incorrect choice of parts for each treatment option.

The described features, structures, advantages, and/or characteristics of the subject matter of the present disclosure may be combined in any suitable manner in one or more embodiments and/or implementations. In the following description, numerous specific details are provided to impart a thorough understanding of embodiments of the subject matter of the present disclosure. One skilled in the relevant art will recognize that the subject matter of the present disclosure may be practiced without one or more of the specific features, details, components, materials, and/or methods of a particular embodiment or implementation. In other instances, additional features and advantages may be recognized in certain embodiments and/or implementations that may not be present in all embodiments or implementations. Further, in some instances, well-known structures, materials, or operations are not shown or described in detail to avoid obscuring aspects of the subject matter of the present disclosure.

### BRIEF DESCRIPTION OF THE DRAWINGS

In order that the advantages of the subject matter may be more readily understood, a more particular description of the subject matter briefly described above will be rendered by reference to specific embodiments that are illustrated in the appended drawings. Understanding that these drawings depict only typical embodiments of the subject matter and are not therefore to be considered to be limiting of its scope, the subject matter will be described and explained with additional specificity and detail through the use of the drawings.
FIG. 1 is a block diagram of elements of an embodiment of the system;
FIG. 2 is a perspective view of a handpiece;
FIG. 3 is a perspective view of the handpiece of FIG. 2 with a portion of the cover removed;
FIG. 4 is a perspective view of the handpiece of FIG. 2 with the cover fully removed;
FIG. 5 further partially cut-away view of the handpiece of FIG. 2;
FIG. 6 is a perspective view of the handpiece of FIG. 2 with the cartridge removed;
FIG. 7 is a perspective view of an oscillation carriage lift interface;
FIG. 8 is an oscillation carriage lift gear control;
FIG. 9 is a perspective view of an oscillation drive;
FIG. 10 is a perspective view of an oscillation drive, a rotation drive, and a cartridge interface;
FIG. 11 is a perspective view of a cartridge element fully assembled;
FIG. 12 is a perspective view of a disposable cartridge;
FIG. 13 is a cutaway of disposable cartridge revealing internal components;
FIG. 14 is an exploded view of a needle holder assembly, and a drive rod;
FIG. 15 is a perspective view of the needle holder assembly of FIG. 14;
FIGS. 16A-E are end views of the needle holder assembly showing embodiments of various needle configurations;
FIGS. 17A-C are elevation side views of various embodiments of needle configurations;
FIG. 18A is elevation side and edge views of an embodiment of a needle configuration;
FIGS. 18B-F are elevation side views of various embodiments of needle configurations;
FIG. 19 is a perspective view of a drive, a connection shaft, and a handpiece;
FIG. 20 is a perspective view of the drive, the connection shaft, and the handpiece of FIG. 19;
FIG. 21 is a perspective view of the drive system of FIG. 19;
FIG. 22 is a perspective view of an alternative view of the drive system of FIG. 21;
FIG. 23 is a perspective view of a connection shaft;
FIG. 24 is a perspective view of a handpiece and a spacing ring;
FIG. 25 is a perspective view of the handpiece and spacing ring of FIG. 24;
FIG. 26 is a cross-section view of the handpiece cartridge and spacing ring of FIG. 24;
FIG. 27 is a perspective view of a disposable cartridge;
FIG. 28 is a cross-section view of the disposable cartridge of FIG. 26;
FIG. 29 is a perspective view of a needle bundle coupled to the disposable cartridge of FIG. 26;
FIG. 30 is a schematic view of elements of the system;
FIG. 31 is a block diagram of a controller;
FIG. 32 is a perspective view of an oscillating motor;
FIG. 33 is a perspective view of a drive system having a rotational drive motor and a flexible shaft coupled to a handpiece;
FIG. 34 is a sectional view through the rotational axis of the drive motor, the flexible shaft and the handpiece of FIG. 33;
FIG. 35 is a perspective view of the handpiece and the disposable cartridge decoupled;
FIG. 36 is a perspective view of the handpiece and disposable cartridge coupled together;
FIG. 37 is a sectional view of the handpiece and the disposable cartridge decoupled;
FIG. 38 is a sectional view of the handpiece and disposable cartridge coupled together showing the mechanical relationships of the parts;
FIG. 39 is a sectional view of the handpiece and the disposable cartridge decoupled;
FIG. 40 is a sectional view inside the handpiece showing the mechanical relationships of the various parts;
FIG. 41 is a perspective view of the handpiece with the handle removed showing the rotational housing and the alignment tabs located on the disposable cartridge;
FIG. 42 is a perspective view of the rotational housing of the handpiece coupled to the disposable cartridge;
FIG. 43 is a transparent perspective view of the internal components of the handpiece with a rotational housing and a rotational drive; and
FIG. 44 is a transparent perspective view of the handpiece illustrating a rotational shaft moved forward toward a proximal end of the handpiece causing displacement of the needle bundle.

It will be appreciated that the drawings are illustrative and not limiting of the scope of the invention which is defined by the appended claims. The embodiments shown accomplish various aspects and objects of the invention. It is appreciated that it is not possible to clearly show each element and aspect of the invention in a single figure, and as Such, multiple figures are presented to separately illustrate the various details of the invention in greater clarity. Similarly, not every embodiment need accomplish all advantages of the present invention.

While the disclosure is susceptible to various modifications and alternative forms, specific embodiments have been shown by way of example in the drawings and will be described in detail herein. However, it should be understood that the disclosure is not intended to be limited to the particular forms disclosed. Rather, the intention is to cover all modifications, equivalents and alternatives falling within the scope of the invention as defined by the appended claims.

Throughout the description, similar reference numbers may be used to identify similar elements.

### DETAILED DESCRIPTION

It will be readily understood that the components of the embodiments as generally described herein and illustrated in the appended figures could be arranged and designed in a wide variety of different configurations. Thus, the following more detailed description of various embodiments, as represented in the figures, is not intended to limit the scope of the present disclosure but is merely representative of various embodiments. While the various aspects of the embodiments are presented in drawings, the drawings are not necessarily drawn to scale unless specifically indicated.

Unless otherwise indicated, the terms "first," "second," etc. are used herein merely as labels, and are not intended to impose ordinal, positional, or hierarchical requirements on the items to which these terms refer. Moreover, reference to, e.g., a second item does not require or preclude the existence of, e.g., a "first" or lower-numbered item, and/or, e.g., a "third" or higher-numbered item.

Additionally, instances in this specification where one element is "coupled to another element" can include direct and indirect coupling. Direct coupling can be defined as one element coupled to and in some contact with another element. Indirect coupling can be defined as coupling between two elements not in direct contact with each other but having one or more additional elements between the coupled elements. Further, as used herein, securing one element to another element can include direct securing and indirect securing. Additionally, as used herein, "adjacent" does not necessarily denote contact. For example, one element can be adjacent to another element without being in contact with that element.

In the above description, certain terms may be used such as "up," "down," "top," "bottom," "upwards," "downwards," "upper," "lower," "horizontal," "vertical," "left," "right," "over," "under" and the like. These terms are used, where applicable, to provide Some clarity of description when dealing with relative relationships. But these terms are not intended to imply absolute relationships, positions, and/or orientations. For example, with respect to an object, an "upper" surface can become a "lower surface simply by turning the object over. Nevertheless, it is still the same object. Further, the terms "including," "comprising," "having," and variations thereof mean "including but not limited to" unless expressly specified otherwise. An enumerated listing of items does not imply that any or all of the items are mutually exclusive and/or mutually inclusive, unless expressly specified otherwise. The terms "a, " "an," and "the" also refer to "one or more" unless expressly specified otherwise. Further, the term "plurality" can be defined as "at least two."

Unless otherwise indicated, the terms "first," "second," etc. are used herein merely as labels, and are not intended to impose ordinal, positional, or hierarchical requirements on the items to which these terms refer. Moreover, reference to, e.g., a second item does not require or preclude the existence of, e.g., a "first" or lower-numbered item, and/or, e.g., a "third" or higher-numbered item.

The schematic flow chart diagrams and method schematic diagrams described above are generally set forth as logical flow chart diagrams. As such, the depicted order and labeled steps are indicative of representative embodiments. Other steps, orderings and methods may be conceived that are equivalent in function, logic, or effect to one or more steps, or portions thereof, of the methods illustrated in the schematic diagrams.

Furthermore, the described features, advantages, and characteristics of the invention may be combined in any suitable manner in one or more embodiments.

Reference throughout this specification to "one embodiment," "an embodiment," or similar language means that a particular feature, structure, or characteristic described in connection with the indicated embodiment is included in at least one embodiment of the present invention. Thus, the phrases "in one embodiment," "in an embodiment," and similar language throughout this specification may, but do not necessarily, all refer to the same embodiment.

While many embodiments are described herein, at least some of the described embodiments allow for the efficient removal of tattoos, permanent makeup, and other indelible mark or pigment on and under the skin, whether they were applied deliberately (as in tattooing) of were acquired naturally (as are pigmented lesions and dermal scarring). While the description herein refers primarily to tattoo removal, the apparatuses, systems, and methods described herein may be also be utilized for tattooing or other treatments to the skin of a client. Two broad classes of treatments that can be accomplished by the embodiments described herein include superficial dermabrasion (for scar revision and for removal of tattoos, permanent makeup, pigmented lesions, etc) and axial needling or aesthetic microneedling (for tattooing, skin tightening, wrinkle removal, etc).

Throughout this disclosure, reference will be made to facilitating fluid and/or Teprsol^{®}, a registered trademark of Rejuvatek Medical Inc. It should be understood that these terms are used synonymously to refer to any appropriate facilitating fluid as would be understood by one of ordinary skill in the art, whether that fluid is Teprsol^{®} or another fluid. The facilitating fluid may be any liquid solution formulated to aid the system in performing its various superficial dermabrasion and/or axial needling procedures, and the formulation may change and be fitted to particular procedures.

References throughout the disclosure to dermal penetrative and shear forces may be generated by either blades, needles, brushes, or similar implements that may engage in three mechanical motions: fast oscillatory axial motions, slow positional axial motions, and slow rotation lateral motions. Note that "fast" and "slow" are relative terms. The term "axial" means substantially along the treatment needle axis, and the term "lateral" means perpendicular or transverse to the treatment needle axis. The system may automate treatment procedures by programming four independent degrees of freedom: the three mechanical brush motions and a facilitating fluid flow.

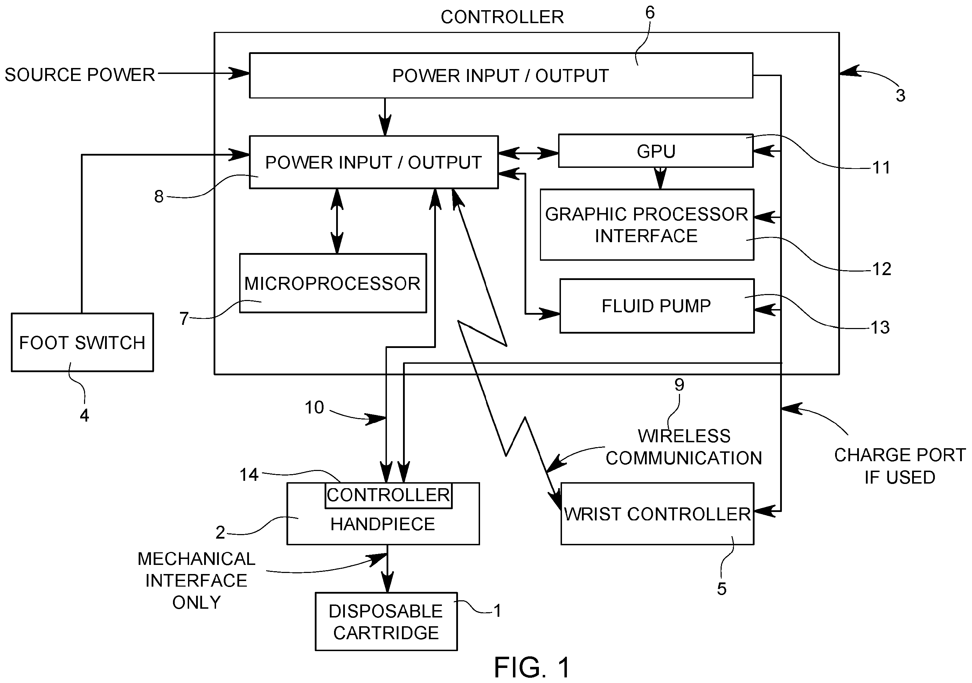

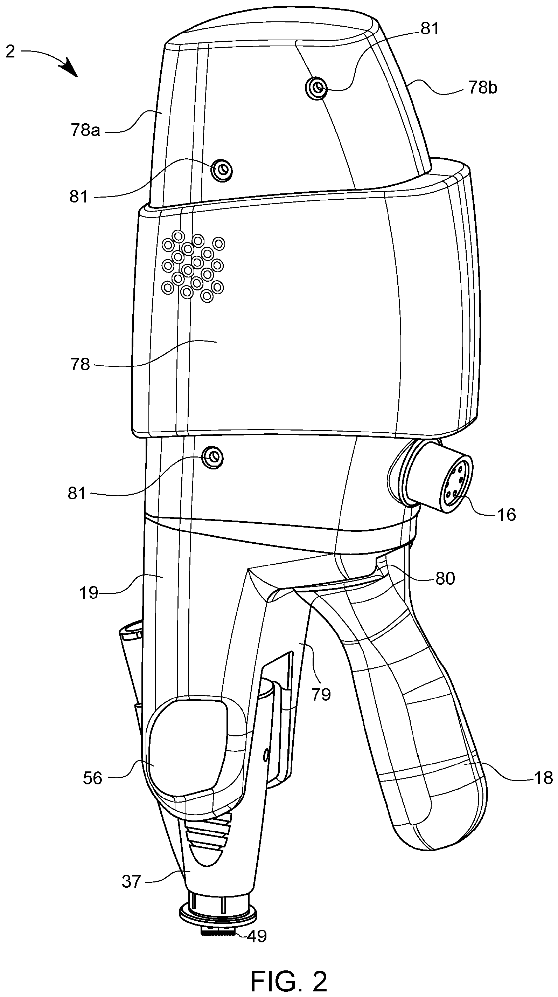

In accordance with embodiments of the system, FIG. 1 illustrates a flow chart exhibiting elements of the system for use in the application and removal of tattoos and other skin treatments comprising a cartridge assembly 1, a handpiece 2, a system controller 3, a foot controller 4 and a wearable wrist controller 5. Each of these elements may be varied in design, component arrangement, materials, and construction without deviating substantially from the functions described herein. The system controller 3 comprises a power input/output 6, wherein the system derives power from an external source such as a wall outlet. The system controller 3 monitors and sets voltage values for the operation of a drive system 15, as shown in FIG. 2, that may be either enclosed within the housing or situated externally. The system controller 3 comprises a microprocessor 7 featuring microprocessor controls, memory storage, operations controls, and monitoring functions. The microprocessor 7 provides instructions to other components in the system controller 3.

The system controller 3 may comprise an input/output ("I/O") section or module 8. The I/O section 8 provides an interface with external components of the system such as connections to the handpiece 2, touch control, power controls and other inputs from the user and outputs to the user. The I/O section 8 also enables wireless communication 9 via a remote-control unit on the wearable wrist controller 5. Moreover, the I/O section 8 processes data communications feedback 10 from the handpiece 2 indicating the correct operation, measurement, and control of the speed of oscillation, measurement and control of the rotation motor, and the position motor feedback location and position. Each component may provide feedback that is input to the microprocessor 7.

The microprocessor 7 interfaces with and controls the various components of the system in accordance with computer code, programming, and instructions that are stored and executed within microprocessor. The microprocessor 7 interfaces with the graphics processing unit 11 that translates instructions and programs into graphical images that can be interpreted by the user for operation of the device. The graphics processing unit 11 communicates with a graphical display on a graphic processor interface 12 with takes instruction sets and presents them in picture form to a user. In response, the user can then react to the picture form and provide input to the microprocessor 7 via the graphics processing unit 11 using the graphic processor interface 12.

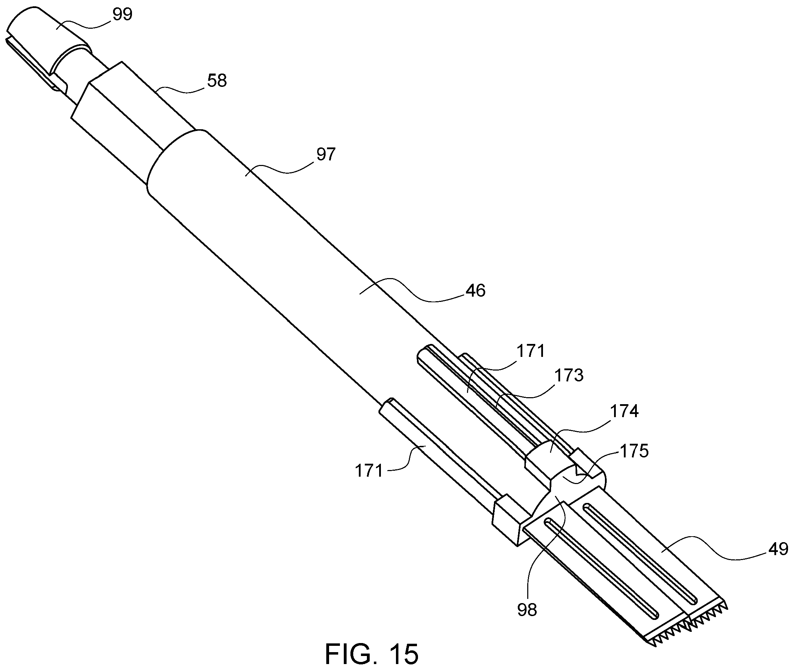

The system controller 3 may further comprise a communications interface. If external devices are attached and are to be controlled, the graphic processor interface 12 provides that interface between various elements including the foot controller 4 or the wearable wrist controller 5 and a fluid pump 13. Other items as listed in the discussions below might include automatic control of the handpiece by optical or switch control operated by the clinician. Communication between components may be provided by various wireless control methodologies such as Bluetooth or Wi-Fi. A handpiece control 14 may control the oscillation, rotation, and depth of a needle bundle 49, as illustrated in FIG. 15. The operation and control of the needle bundle 49 controls the interaction at the surface of the patient's skin where treatment occurs.

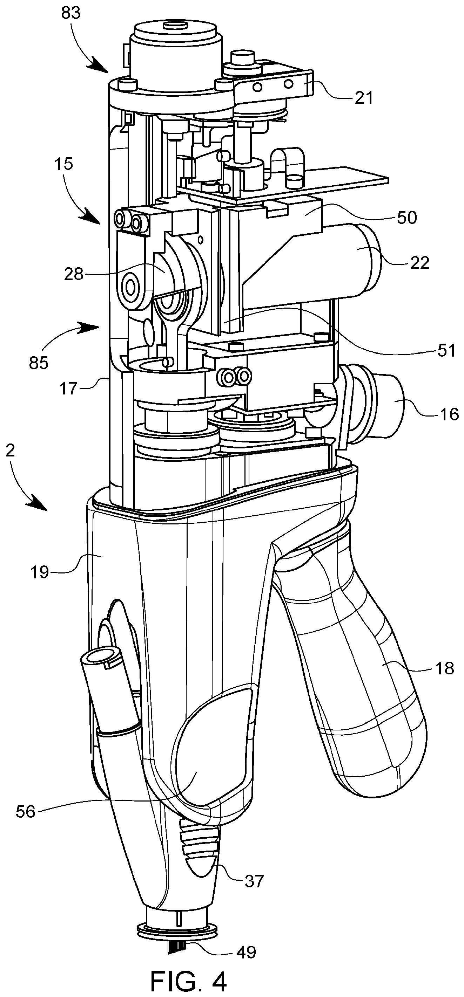

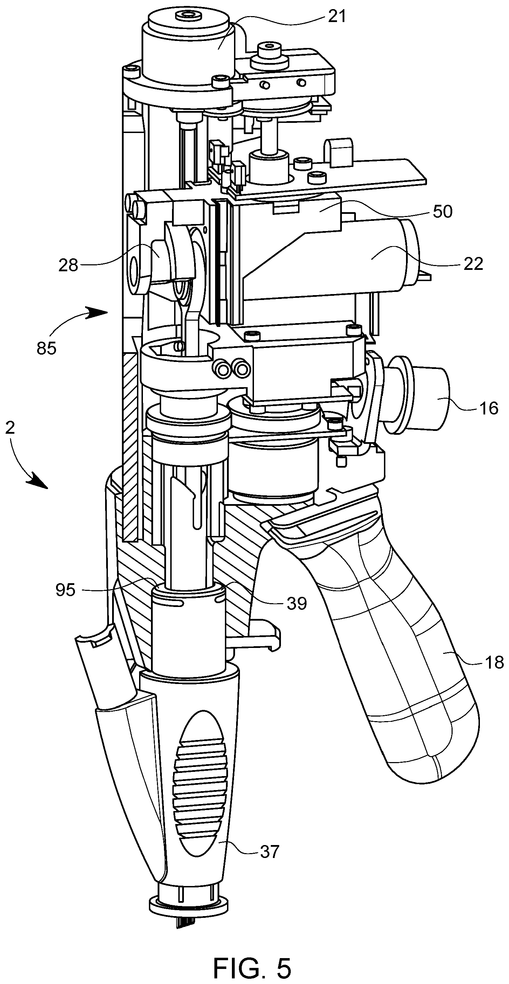

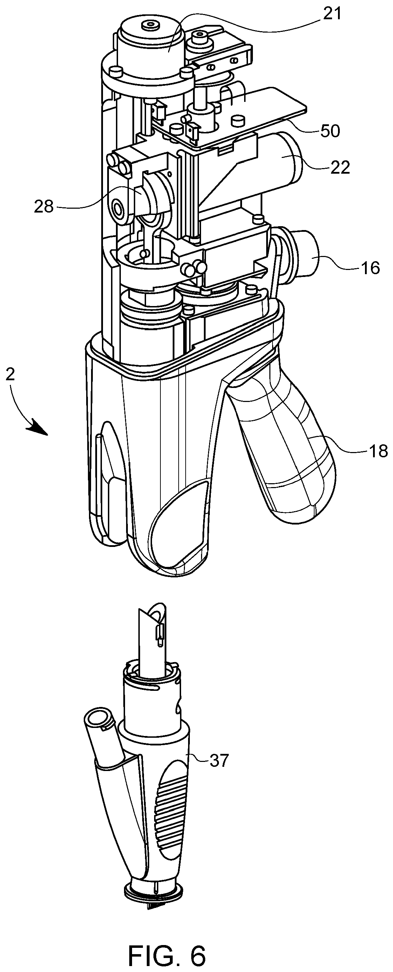

As illustrated in FIGS. 2-6, embodiments of the handpiece 2 may comprise a housing 78. The housing may include first and second housing portions 78a, b that are connected with each other and that provide protection and containment for internal components. Housing portions 78a, b may be removable as necessary for maintenance and reassembled as needed to perform their function. Features may be formed on an outer surface of the housing 78, such as grip elements or ventilation holes 84. The handpiece 2 is couplable to the controller via a flexible cable routed to a connector 16 on the handpiece 2. The connector is connected to an internal handpiece controller 14, that communicates with the I/O section 8 of the microprocessor 7 and provides control to each system within the handpiece 2. Sensor and motion controls may be routed through the controller 14 of the handpiece 2 which is connected to each sub system.

Embodiments of the handpiece 2 may comprise a handle portion 19. Attached to a handle 19 may be an auxiliary grip 18 that can be attached to or adjacent a lower surface 79 of the handle. The Auxiliary grip 18 may be attached by a dovetail mating feature 80 or other appropriate connection. This auxiliary grip 18 is held as a stabilizer when the thumb and pointer finger of the technician grip the handle 19 in one or more recesses 56 that may be positioned near an end of the handle portion 19 adjacent to a disposable cartridge 37. The auxiliary grip 18 extends into the technician's hand and may be semi-gripped by the remaining fingers of the hand.

Embodiments of the handpiece 2 may comprise a vertical frame 17 that holds or connects various components of a drive system 15 in position and alignment. Fasteners 81 may extend through the first housing portion 78a to the second housing portion 78b. Fasteners 81 may extend through or connect with apertures 82 in the vertical frame 17 positioned in an interior cavity created at least in part by the first and second housing portions 78a, b. In exemplary embodiments, on the distal end or top of the frame 17 is a block 21 that holds a lift mechanism 83 of the drive system 15 in place.

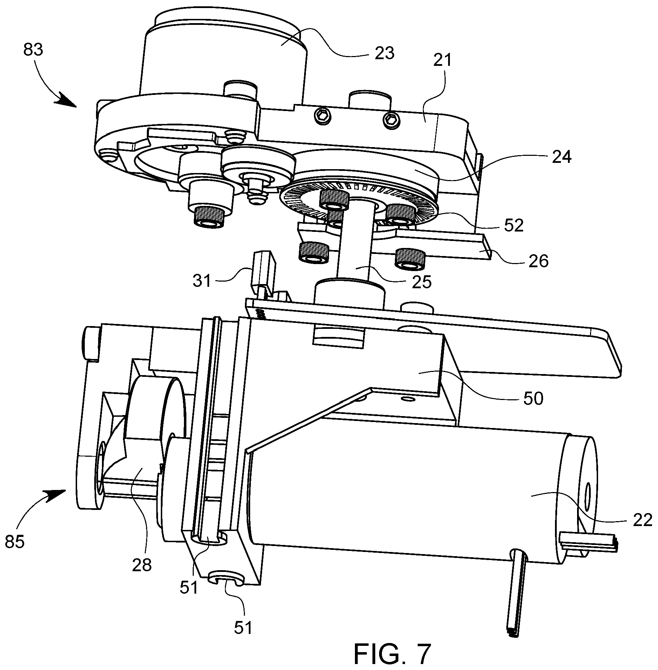

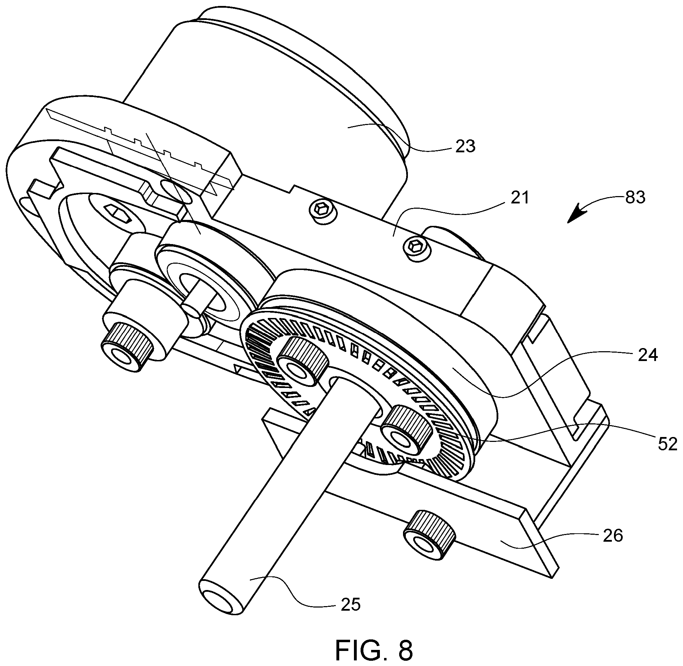

Embodiments of the lift mechanism 83 are illustrated more fully in FIGS. 7-8. Attached to the block 21 is a gear train 24 which rotates a lead screw shaft 25. The gear train 24 drives other gears that are attached to a first stepper motor 23. The first stepper motor 23 provides rotational motion to the gear train 24 that interfaces with the lead screw shaft 25. The rotational motion of the first stepper motor 23 rotates the lead screw shaft 25 which is connected to an oscillation drive frame 50, thereby positioning the drive on the vertical frame 17 in the position set by the system controller 3. Linear bearings 51 are fitted to the carriage to allow it to smoothly be positioned as needed. The distance traveled or the position with regards to height is measured by a slotted signal ring 52 and read by sensors on a printed circuit board 26. The limit of travel for the oscillation drive frame 50 is measured by hall effect devices 31 that may be mounted on the oscillation drive frame 50. At each end of travel are magnets coupled to the vertical frame 17 that communicate with the hall effect devices 31 thereby setting the limits of travel or setting position. Power for the first stepper motor 23 is sent from the handpiece controller 14 that receives motor voltages through the flexible cable connected at the connection shaft assembly 16.

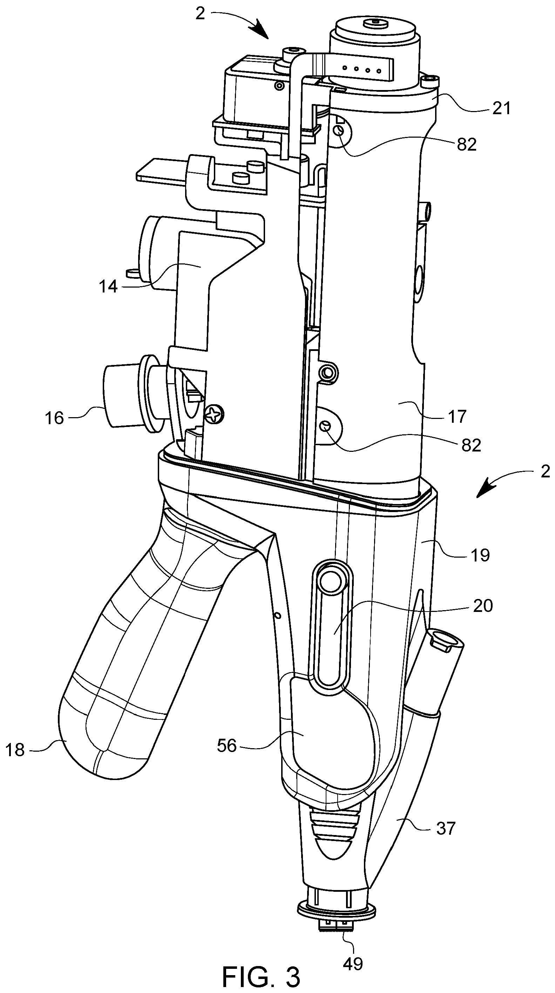

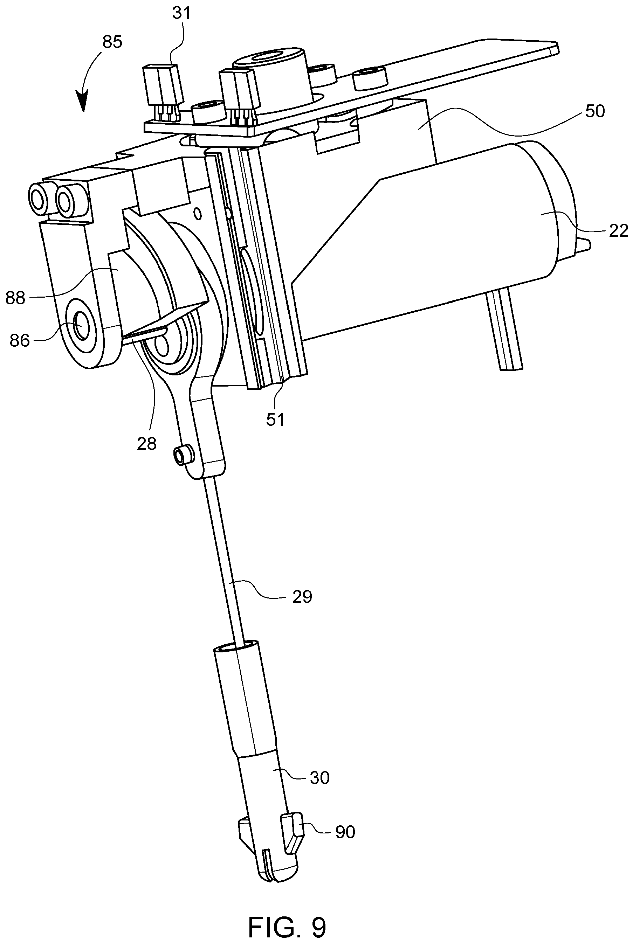

Returning to FIGS. 3-6, embodiments of the drive system 15 may also comprise an oscillation drive 85. This drive 85 may comprise an oscillation motor 22 that is speed controlled with signals from the microprocessor 7 that are transmitted first to the handpiece controller 14 and then to a brushless motor commutator. As further illustrated in FIGS. 9-10, on an end of the shaft 86 of the oscillation motor 22 is an eccentric crank 28 that is rotated by the oscillation motor 22. Coupled to the eccentric crank 28 is a wire rod 29 that extends out from the eccentric crank 28 and through a rotational block 34 and interfaces with a drive rod 30 within a drive rod 38 of the cartridge. When the wire rod 29 is held in an aperture 87 of the rotational block 34, rotational motion is reduced to a linear motion. This linear motion is used to drive the needle bundle 49 in a linear motion to impinge the tissue of the recipient when the needle bundle 49 is placed against the treatment site tissue.

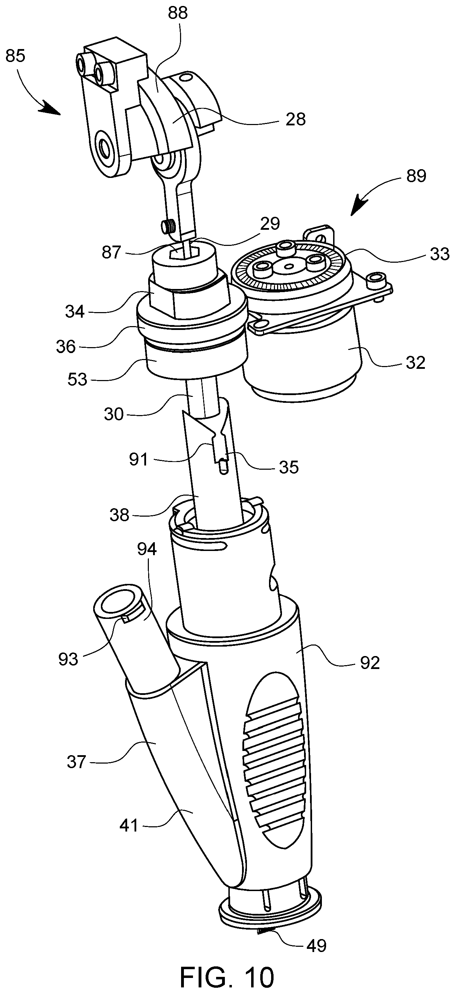

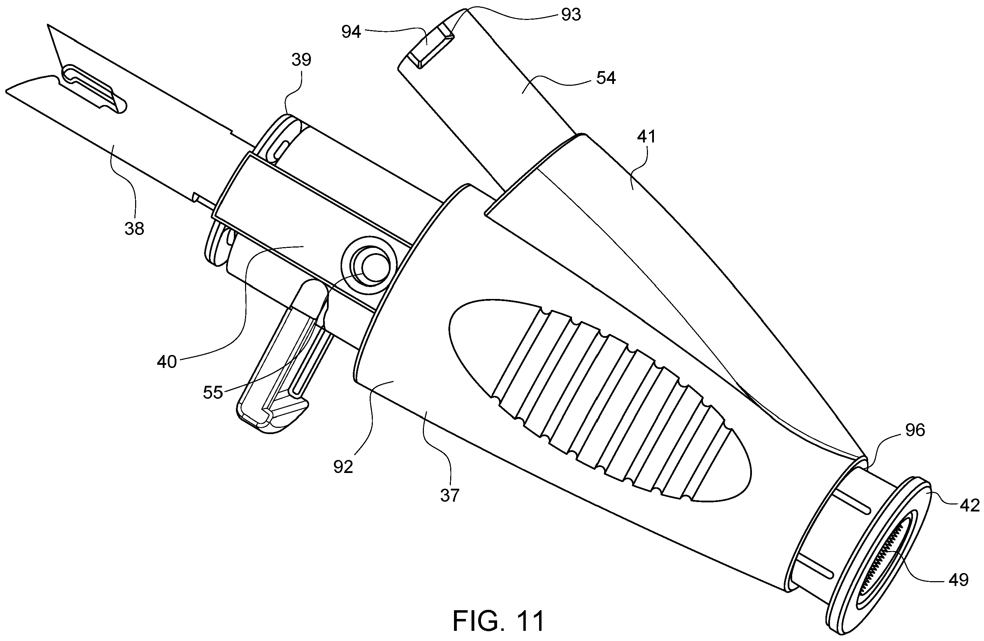

The oscillation motor 22 is mounted in the oscillation drive frame 50 that can be moved in a linear motion due to its riding the vertical frame 17 and sliding on the linear bearings 51. The oscillation drive frame 50 may be moved and positioned anywhere within a specified range controlled by the hall effect devices 31 and magnets mounted on the vertical frame 17. This vertical motion allows the needle bundle to be positioned with the tips even with the cartridge end or projecting a specific amount as determine by the treatment parameters. (See FIGS. 10-11.) Counterweights 88 on the eccentric crank 28 reduce the vibration from the oscillation motor drive to make operation comfortable to the technician.

FIG. 10 illustrates a relationship between embodiments of the oscillation drive 85 and the cartridge 37. This figure also shows an embodiment of an additional drive 89 which causes rotation of the drive rod 30 which transmits that rotational motion to the drive rod 38 on the cartridge. Embodiments of this drive impart rotational motion as described in the operational discussion to the needle bundles. This rotational motion may replace hand motion performed by the technicians during the abrasion process. Rotational motion is started by driving a second stepper motor 32 which transmits that motion to a gear combination 36. The gear combination is secured to the rotational block 34 that is suspended in radial bearings 53. When the second stepper motor 32 is rotated, the rotational block 34 also rotates at a 1:1 ratio. The center of the rotational block 34 has a hexagonal aperture within its center. The drive rod 30 is fitted into the block and has a similar hexagonal form to it. When the rotational block 34 is rotated, the drive rod 30 rotates. The amount of rotation and the speed of rotation is measured by sensors positioned to look at a signal wheel 33. When the rotational signal is sent from the microprocessor, the handpiece controller 14 receives that signal and provides direction and speed information to the second stepper motor 32 to rotate the rotational block 34. The cartridge drive rod 38 comprises an interface slot 35 that transmits the motion received from the drive rod 30 to the cartridge drive rod 38.

FIG. 9 also shows male interface features 90 that are used to transmit motion from the drive rod 30 to the cartridge drive rod 38 in embodiments of the drive system 15. These interface features 90 may comprise tabs extending from an exterior surface of the drive rod 30 to engage with corresponding slots 91 formed in the cartridge drive rod 38. Since the oscillation motion and the rotational motion are controlled separately and merged into the rotational block and drive rod assembly, both motions are independently controlled and can be merged into compound motions of linear and rotational motion which is transferred to the needle bundle 49.

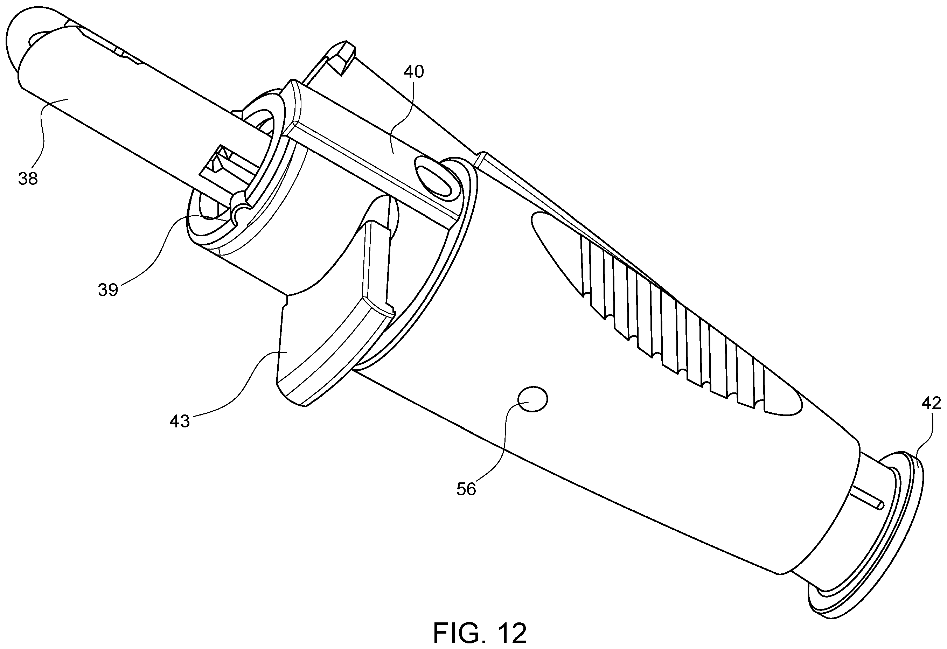

FIGS. 10-14 illustrate embodiments of the disposable cartridge 37. The disposable cartridge 37 may comprise several molded parts assembled together and sterilized prior to use. A housing 92 of the disposable cartridge 37 may be made from a molded plastic material that is durable and that can be sterilized. The molding may comprise polycarbonate, an ABS/polycarbonate blend, or similar polymer. The housing may comprise a protruding side 41 comprising a locking feature 93. The locking feature may comprise tabs or extensions 94 that are adapted to engage female threads formed on an inside surface of an engaging luer lock. The locking feature enables tubing sets that transmit fluids to traverse through the joints with little chance for leakage. Within the locking feature is a small bore 44 that enables fluid that is introduced at the locking feature to be transmitted down to the end of the cartridge at the end of the needle bundles, where the abrasion or treatment takes place. A smaller integral aperture 55 is added to the cartridge passage to limit the flow of the Teprsol^{®} fluid during operation. This will prevent free flow of the fluid onto and around the treatment site possibly wasting fluid or allowing too much fluid to be released during treatment.

In further embodiments, the housing also has upon it raised features 40 that ensure that the disposable cartridge 39 can be only installed one way and is secure during operation. A recess in the raised feature interfaces with a retainer button 20 located on the handle 19 which securely holds the cartridge during treatments. (See FIG. 3.) Once locked in place the vibration that occurs during use will not be sufficient to cause these parts to separate during the treatment. On the distal end of the cartridge is a molded spring 39 that presses against a disposable cartridge mount surface 95 in the handpiece and the spring pressure keeps the disposable cartridge 37 from vibrating during use. This spring force also enables the disposable cartridge 37 to be ejected following treatment, with an advantage of reducing the chance of the technician accidentally contacting the needles/blade.

The disposable cartridge 37 may also comprise a cylindrical feature 96 on the distal end that is the location for the attachment of a spacer ring 42. The tolerances of the cylindrical feature and the spacer ring 42 together allow the two to be mated and remain coupled throughout the treatment cycle. The treatment ring 42 may help the technician space the tegula one from another to allow the skin bridge remaining between tegulae to remain and promote correct healing. The protruding side 41 on a top of the disposable cartridge 37 is the location of a flexible tubing connection 54. The flexible tubing connection 54 is a connection for a tubing set to be attached which allows the pump and fluid dispensing to be located several feet away from the treatment site. Within the proximal end of the disposable cartridge 37, the needle bundle 49 is shown seated within and protected from damage by the cylindrical flange surrounding the needle bundle 49.

The smaller integral aperture 55 on a passage of the disposable cartridge 37 limits the flow of the Teprsol^{®} fluid during operation. This will prevent free flow of the fluid onto and around the treatment site possibly wasting fluid or allowing too much fluid to be released during treatment. The disposable cartridge 37 further comprises a spring 45 that assists the needle bundle 49 to retract during the treatment process. The retraction may ensure that the needle bundle 49 can rotate freely during the treatment. Rotation of the needle bundle 49 may occur before total retraction of the needle bundle 49 if desirable to do so. Alternatively, the disposable cartridge 37 may not comprise a spring, or the spring 45 may serve only to ensure that the assembly internal components are maintained in a proper position. In such embodiments the drive mechanism, including the drive rod 38, may directly drive the needle bundle rod 46 in extension and retraction such that the reciprocating motion does not depend on the action or return force of the spring 45.

A seal 47 that prevents excess Teprsol^{®} fluid from exiting to the back of the disposable cartridge 37. Any excess fluid that enters this portion may exit the chamber through an aperture 56. At proximal end of the disposable cartridge 37, a needle interface rod 38 provides interface with components of the handpiece 2 for linear as well as rotational motion. The components of the handpiece 2 travel in a linear motion that directly moves the cartridge drive rod 38 in a linear travel, thus moving a needle bundle rod 46 in a linear travel. When placed against the skin of the recipient, the needle bundle 49 when acted upon in a linear motion through the cartridge drive rod 38 and the needle bundle rod 46, will extend from the distal end of the disposable cartridge 37 and pierce the skin of the recipient.

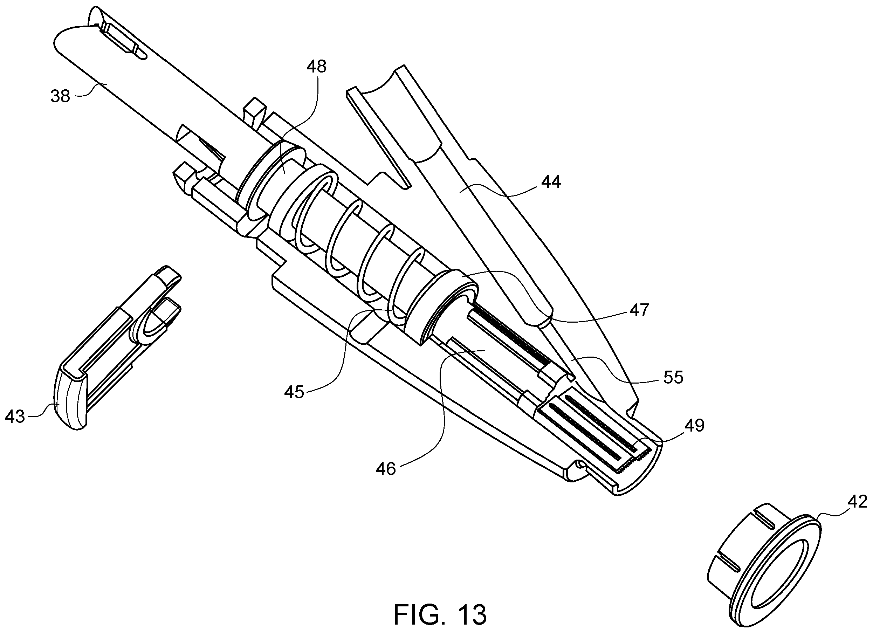

FIGS. 13-14 illustrate embodiments of the needle bundle 49. The needle bundle 49 may be sized larger in diameter or smaller with a high density of needles or a low density of needles being spaced closely or sparsely depending upon the treatment type being performed. It is contemplated that a needle bundle may be designed in other patterns other than circular, such as triangular mount or square mount with various needle patterns on them along with various densities of needles. The embodiment of FIGS. 13-14 shows an offset linear pattern. Other patterns would be understood by one of ordinary skill in the art. The needle holder or bundle rod 46 supports the needle bundle 49 and provides the interface of the needle bundle 49 to the drive components along with strength to press needles into the skin of the recipient. The needle bundle rod 46 may be molded from a plastic material while the needles may be machine inserted and molded in place or hand inserted and molded in place. For sparse needle configurations, the needle bundle rod 46 may comprise holes with the needles hand placed and secured with adhesive.

Embodiments of the needle bundle rod 46 interface with the cartridge drive rod 38 within the housing of the cartridge and are secured by a snap fit. The rear of the needle bundle rod 46 has a hexagonal feature 58 that ensures that the two parts when assembled will be able to be rotated as one part. The needle bundle rod 46 comprises a hexagonal interface feature 58 that interfaces with a mating feature 57 of the cartridge drive rod 38. The hexagonal interface features 58 mating together ensures that rotational motion will be translated to needle bundle.

Embodiments of the cartridge housing further comprises a locking tab 43 which locks the linear travel of the needle bundle rod 46 and the cartridge drive 38. When inserted into the cartridge housing 43, the tab is inserted into an annular recess 48 in the cartridge drive rod 38. This prevents the needles/ blades from extending during installation of the cartridge into the handpiece. It is removed after installation of the disposable cartridge 37 and can be discarded or retained for reinstallation when the disposable cartridge 37 is ready to be ejected and discarded.

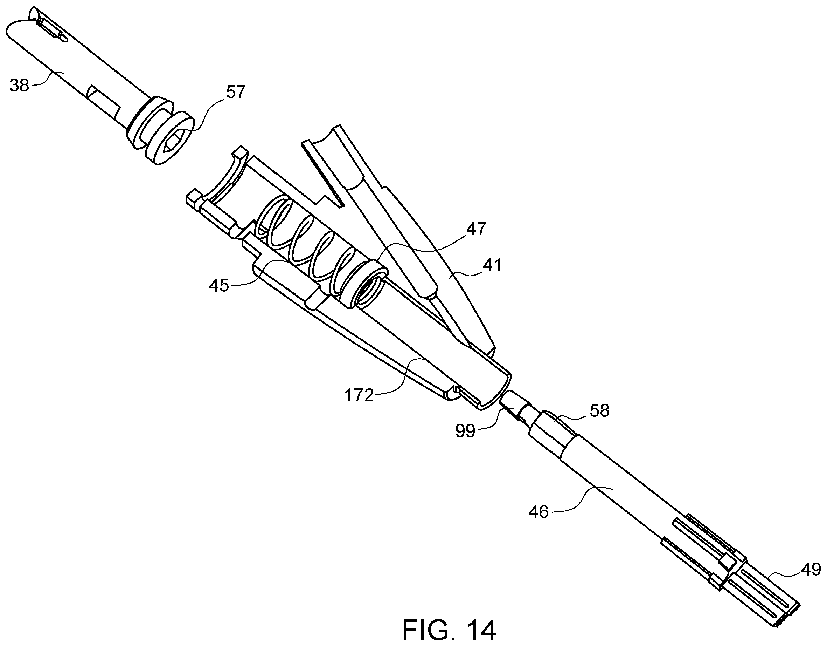

As illustrated in FIGS. 14-15, the needle holder may include a generally cylindrical shaft 97 that extends from a needle face 98 at a distal end of the holder to the hexagonal interface 58 at the proximal end. Locking tabs 99 or another locking feature may extend from the proximal end of the interface 58. The locking tabs 99 may engage corresponding features on or adjacent to an end of the mating socket 57 of the cartridge drive rod 38. Ridges 171 may extend from a sidewall of the shaft 97 to engage an interior surface 172 of the disposable cartridge. The ridges 171 may comprise a first, proximal section 173 and a second, distal section 174. The distal section may extend farther from the shaft 97 surface and may have an end surface 175 that is coplanar with the needle face 98.The shaft 97 is illustrated as cylindrical but may, alternatively, have a triangular, rectangular, obround, or other cross-sectional shape.

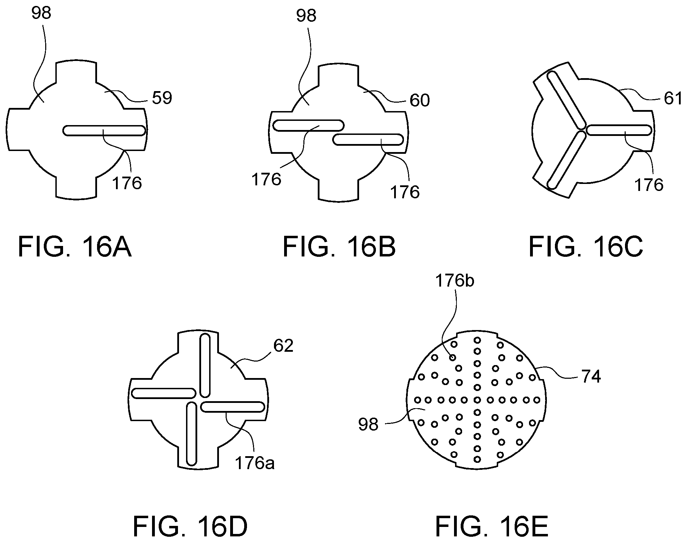

As illustrated in FIGS. 15 and 16A-E, one or more needle bundles 49 may extend from the needle face 98. Needle bundles 49, blades, or individual needles may be attached at the needle face. For example, a needle bundle may be inserted into a recess 176 formed in the needle face. The needle bundle may be attached by inserting the needle bundle into the recess and securing it by adhesive, press fit, or other mechanisms. Alternatively, the needle holder 46 may be injection molded around the needle bundle so that the needle bundle is secured to the holder. The recesses 176 may be elongated 176a, circular 176b, or some other shape depending on the shape of the needle bundle, blade, or needle to be inserted.

Embodiments of the needle holder 49 are illustrated in FIGS. 16A-E, including various combinations and arrangements of needles or blades. For example, the needle packs can be arranged as a singlet 59, a couplet 60, a triplet 61, or a quadruplet 62. If single needles are desired for certain types of applications, the single needles can be grouped or spaced in a grid or pattern configuration 74 having a plurality of needles in a grid formation extending outwards from a center region. Alternatively, a needle holder may be provided with a series of recesses 176b arranged in a pattern 74 on the needle face 98 into which a user can place one or more needles in a custom arrangement suited to the user's need or preference. It will be appreciated that the potential arrangements can vary depending upon the application for the cartridge and what positions are filled in the needle holder.

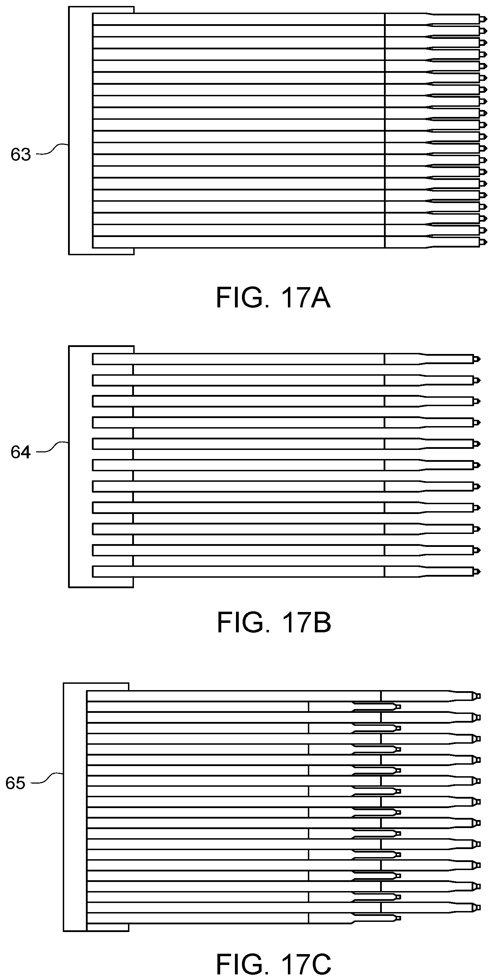

FIGS. 17A-C illustrate elevation views of embodiments of needle/blade configurations. A compact linear configuration 63 may orient a group of needles in a tight single layered arrangement. Such groupings can be installed in the recess 176 in the needle face 98 with various arrangements depending on the molded portion. Alternatively, a spaced linear configuration 64 may orient a group of needles spaced apart from one another without touching or overlapping needles. A staggered configuration 65 may orient a group of needles in two or more rows with the needles in a first row offset spatially from the needles in a second row. The needles in the second row may similarly be offset from needles in subsequent rows.

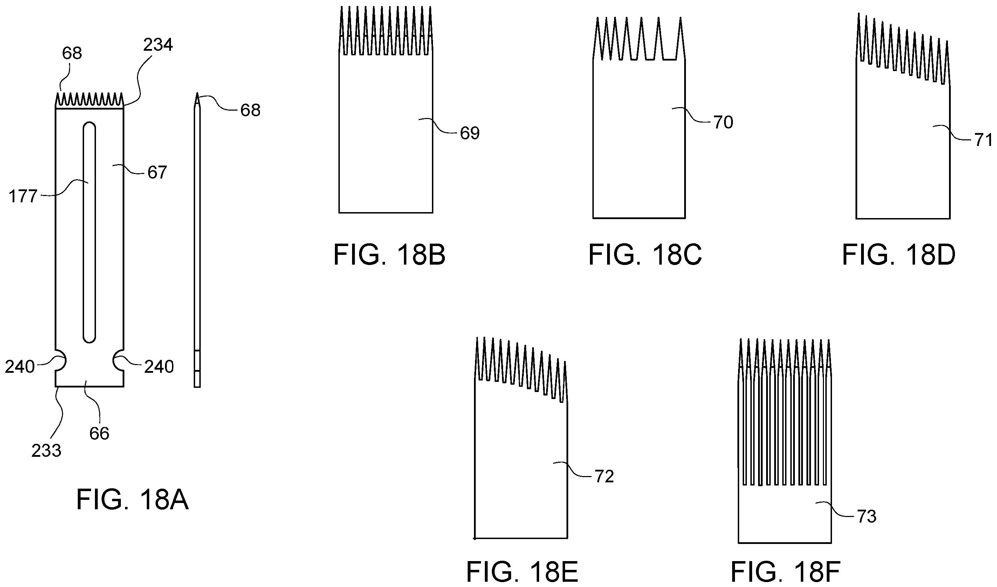

FIGS. 18A-F illustrate elevation views of embodiments of needle assemblies having various configurations. A needle assembly 66 may comprise a securement end 233 and a penetration end 234. The securement end 233 may comprise one or notches 240 that aid in securing the needle assembly to the needle holder 46. The penetration end 234 may comprise one or more individual needles or a plurality of blades or points 68. The tips of the plurality of blades 68 may be cut with laser energy or may be manufactured from chemical etching processes or similar techniques. A recess or cavity 177 may extend into or through the blade 67. The cavity may reduce the mass or improve the strength, rigidity, or stability of the blade 67. The needle assembly 66 may comprise a flat surgical material that is cut or etched at an angle in one direction to cut a needle or sawtooth shape and ground in the other direction to achieve a sharp needle like shape.

The plurality of blades 68 may feature a uniform spaced configuration 69 in a single row. The plurality of blades 68 may be manufactured by similar means as razor blade processing, creating the taper of the tips with a grinding process to achieve a sharp edge having deeper roots on the blade tip spacing. This allows the plurality of blades 68 to penetrate deeper without the root of the plurality of blades 68 striking the surface of the tissue being impinged. A variable spacing configuration 70 may be created using mathematical spacing calculations such as logarithmic or geometric spacing algorithms. Such spacing may aid in the abrasion as the blade is oscillated linearly while being spun on the axis. An angled configuration 71 may orient the blades at varying heights along a linear axis. Similarly, a curved angle configuration 72 may orient the blades at varying heights along a curved axis. Since the design of the plurality of blades 68 is not limited to a mechanical hand assembly, the design of the plurality of blades 68 has many more options available for fitting other applications in addition to just tattoo removal. For example, a deep recess configuration 73 may comprise recesses along a longitudinal axis between shafts of the plurality of blades 68 similar to needles. The deep recess configuration 73 is conducive to allowing the needles or plurality of blades 68 to be more flexible. Additionally, the spacing may help retain fluids such as ink or pain management analgesics. The deep recess configuration 73 may also be used for artistic tattoo applications or for permanent makeup applications. FIGS. 18A-F illustrate embodiments of the blade 67 having a generally flat, linear configuration formed from a single piece. However, the blade may also be bent or otherwise shaped into other configurations such as a "C," "N," "O," "W," or other shape. Alternatively, the blade may be formed from multiple pieces to form a multi-leg shape, such as a "Y" or "+" shape.

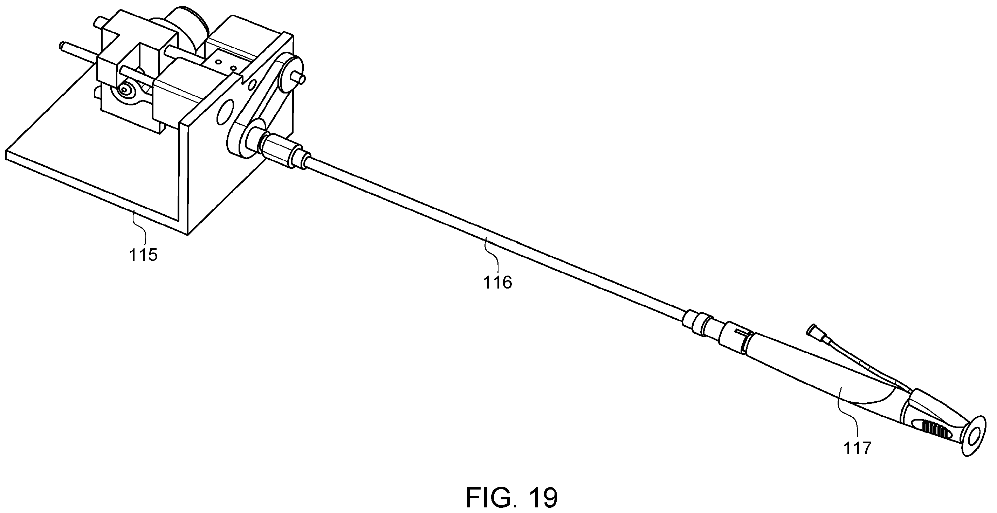

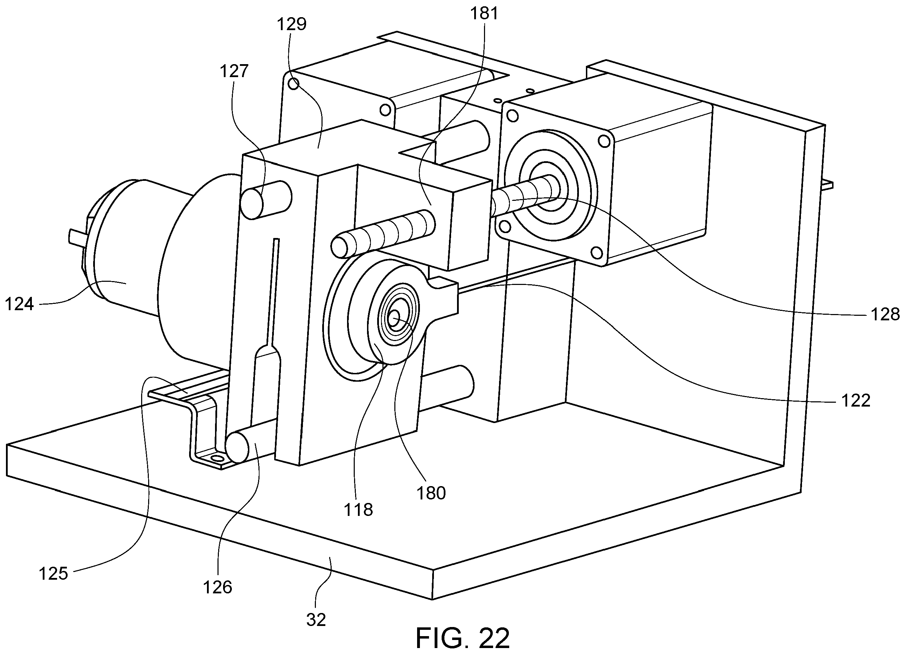

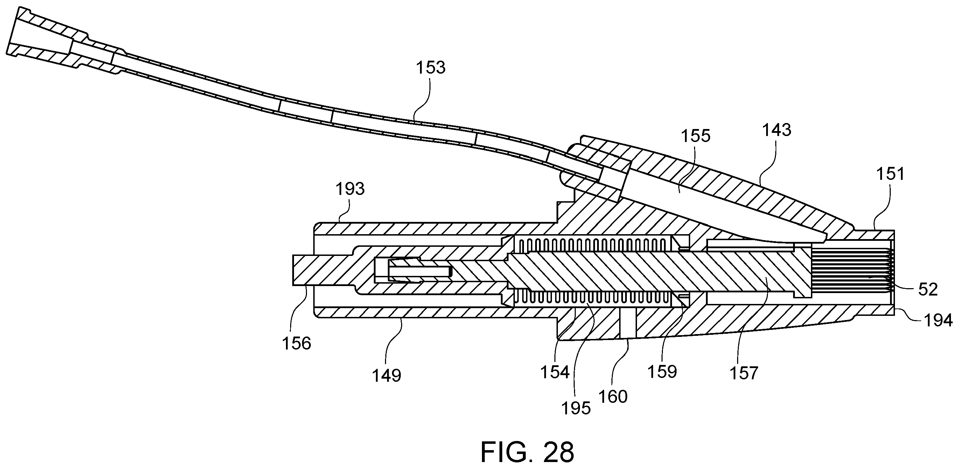

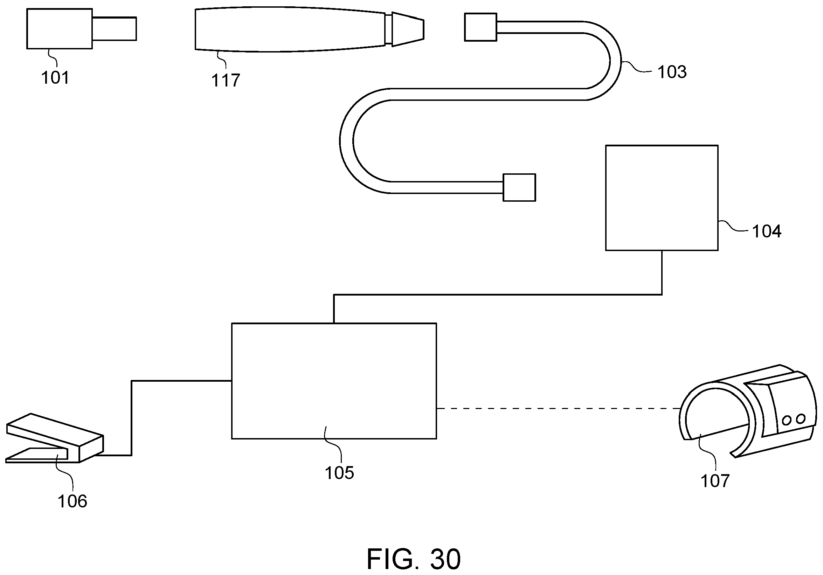

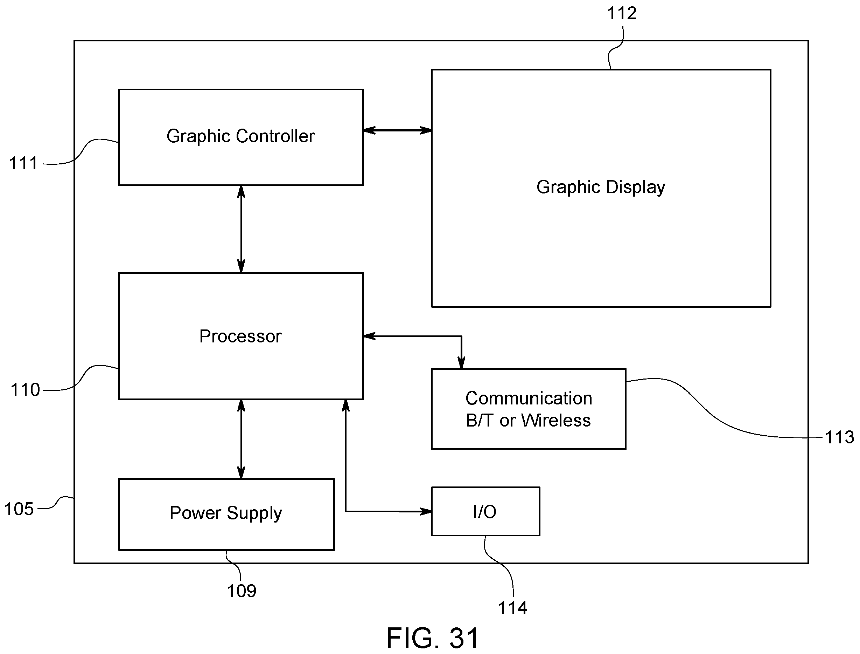

As illustrated in FIG. 30, embodiments of a system comprise a cartridge assembly 101, a hand piece 117, a drive connection 103, a drive 104, a system controller 105, a foot controller 106, and a remote controller 107. As illustrated in FIGS. 19-29, the cartridge assembly 101 may comprise a disposable cartridge 143 comprising a needle bundle 152. The drive connection 103 may comprise a connection shaft assembly 116; and the drive 104 may comprise a drive system 115. Embodiments of the system controller 105, as seen in FIG. 31, may comprise a power supply 109 that obtains power from external sources such as wall outlets. The power supply 109 controls and sets voltage values for the operation of the drive system either enclosed within the housing or externally. A processor 110 of the controller may contain microprocessor controls, memory storage, operations controls, monitoring functions and provides instructions to the other components in the control system. One output of the processor 110 section may be to provide I/O control 114.

I/O control 114 may provide an interface with the external components of the system such as connections to the drive, touch control, power controls and other inputs from the user and outputs that the user can operate with. One of the I/O control 114 functions may be enable user control by the remote controller 107, which may be a wrist controller. Another I/O control 114 function may be to provide feedback from the drive 104 indicating correct operation and measurement and control of the speed of oscillation, measurement and control of the rotation motor, and the position motor feedback location and position. Each of these parts has feedback that is fed to the processor 110 and the processor 110 maintains control based upon the programs and instructions that are stored and executed within.

The processor 110 interfaces with a graphic controller 111 that may function to translate instructions and programs into graphical image that can be interpreted by the user for operation of the device. The graphic controller 111 communicates with a graphic display 112 which takes instruction sets and presents them in picture form. The user can then respond and react to the graphic format and provide input to the processor 110 via the graphic display 112 and its interface component the graphic controller 111.

The system controller 108 may also comprise a communications interface 113. Communications within the system controller 108 is internal and linked by software or hardware controls. If external devices are attached and are to be controlled, the communications interface 113 may provide that interface. Interface with various elements may include a switch on the foot control 106 or other elements on the remote wrist controller 107. Other components or functions may include automatic control of the handpiece 117 by optical or switch control operated by the technician. Various wireless control methodologies such as Bluetooth, or Wi-Fi may be used such that the control system need not be tethered to its parts, such as the wrist controller 107 or the foot controller 106.

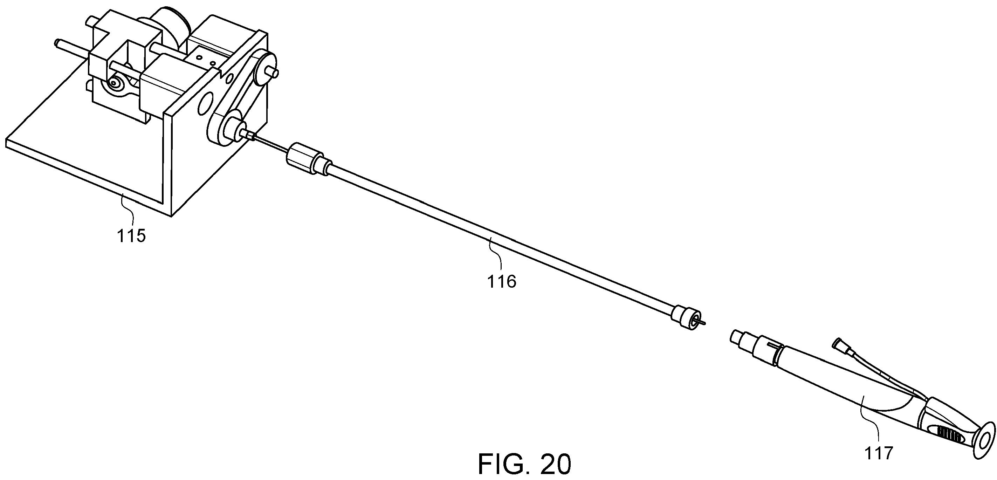

In some embodiments, as shown in FIGS. 19-20, the device may comprise a drive system 115, a connection shaft assembly 116, and a handpiece 117. The drive system 115 may control the oscillation, rotation, and depth of a needle bundle. The connection shaft assembly 116 transfers driving motion from the drive system 115 to the handpiece 117 assembly, to which is attached a cartridge housing that contains the needle bundle. As illustrated in FIG. 20, the drive system 115, connection shaft assembly 116, and handpiece 117 may be separated for construction as well as use and maintenance. These items can be separated if necessary and reassembled as needed to perform their function. The separation allows for different assemblies to be substituted as needed for repair or for upgrade of function.

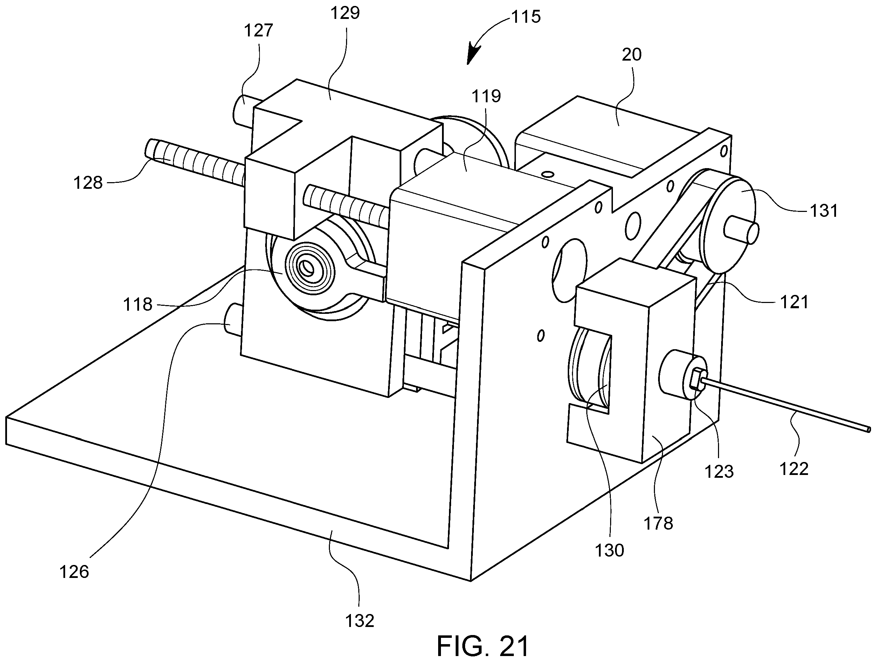

Referring to FIGS. 21-22, the drive assembly 115 may comprise various subsystems, including an oscillation drive, a rotational drive, and a distance or depth drive. In embodiments of the system, a frame 132 holds the various components in position and alignment. Embodiments of the system may comprise a rotational drive in which on the distal end of the frame 132 is a holding block 178 that holds a driven pulley 130 in position. A belt 121 may connect the driven pulley 130 with a drive pulley 131. A stepper motor 120 may rotate the drive pully 131 that, in turn, rotates the driven pulley 130 by means of the belt 121. A drive interface block 123 may be connected with the driven pulley 130 such that the interface block rotates at the pulley rotates. The interface block 123 may engage with the connection shaft assembly 116. The rotational motion is transmitted by the connection shaft, ultimately to the needle bundle. The stepper motor 120 receives instructions from the processor 110 either through a hard connection cable or by wireless communication. Power for this motor is provided by the power supply 109 as are all the motors within the drive sub-system.

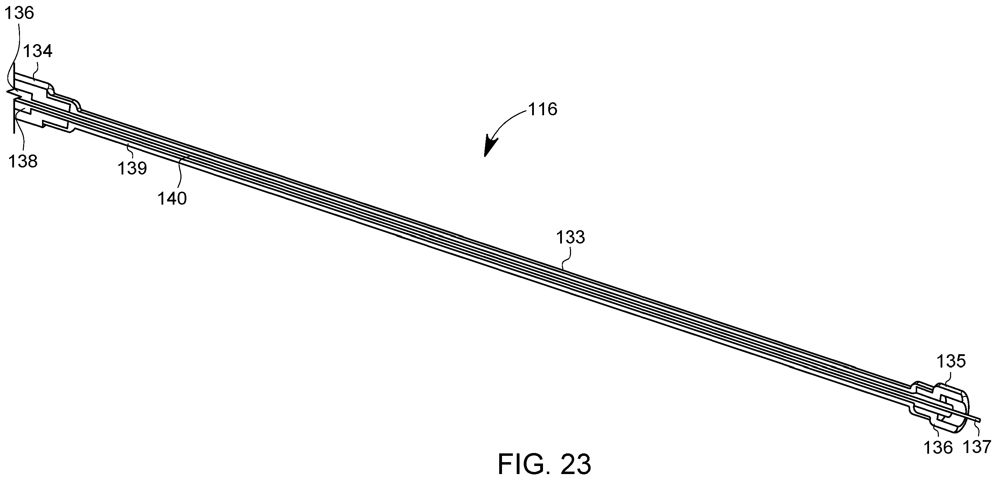

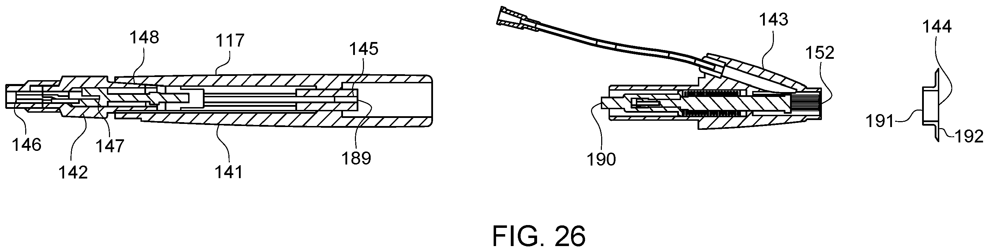

Embodiments of the drive system may also comprise an oscillation drive. The oscillation drive may comprise an oscillation motor 124 that may be a brushed or brushless DC motor that is speed controlled by the processor 110. On the end of the shaft 180 of the oscillation motor 124 is an eccentric crank 118 that is rotated by the oscillation motor 124. Attached to the crank is a wire rod 122 that extends out from the crank and through an aperture in the pulley 130 and interfaces with a flexible rod 140 within the flexible connection shaft 134 (see FIG. 23). When the flexible rod 140 is held in an aperture, the rotation motion is reduced to a linear motion. This linear motion is used to drive the needle bundle 152 in a linear motion to impinge the tissue of the client when the needle bundle 152 is placed against the treatment site tissue (see FIG. 26).

Embodiments of the system may comprise a depth drive in which the oscillation motor 124 is mounted in a carriage 129 that can be moved in a linear motion due to its riding on two control rods 126, 127. The carriage can be moved and positioned along the length of this set of control rods. The drive for this position is provided by a depth stepper motor 119 that has attached to it a threaded rod 128. The carriage 129 has an aperture 181 with an interior female thread that interfaces with the threaded rod 128.

Thus, when the depth stepper motor 119 is repositioned, the carriage 129 is moved either toward the motor 119 or away from the motor. The position of the carriage 129 is monitored by an optical encoder 125 and sensor. The grating of the encoder can allow position of the carriage 129 to be monitored. If desired the motor 119 may incorporate an internal encoder to monitor position of the carriage. As with all other drive components on the drive sub-system, power is obtained from the power supply 109 located within the system controller 108 and are monitored and controlled by the processor 110. Graphical representations of the drive status may be displayed on the graphical display 112, to indicate to the user correct and desired operation of the components.

As illustrated, for example, in FIGS. 23 and 30, the connection shaft assembly 116 may comprise a flexible shaft that can allow positioning of the components within the treatment area of the clinic as well as provide freedom of motion and travel for the technician when placing the handpiece 117 and a cartridge 143 on a client's body. Embodiments of the connection shaft, as shown in FIG. 23, comprise a flexible outer sheath 133 that keeps the items within contained and clean from dust and debris. A proximal end 138 of the flexible connection shaft 134 interfaces with the drive sub assembly 115 and the distal end interfaces with the sub assembly of the handpiece 117. Within the shaft are a flexible tube 139 and a flexible rod 140. The flexible tube 139 has on each end an interface receiving feature 136 that interfaces with the interface block 123 of the drive assembly 115 and a rotational interface feature 146 on the handpiece 102. The interface features 123, 136 transfer rotational motion from the drive assembly 115 to the handpiece 117. The ends of the flexible connection shaft 134 may comprise threaded features or quick disconnect features 135 that secure the ends of the shaft at the drive and handpiece ends. Within the center of the flexible tube 139 inside the flexible outer sheath 133 is housed the flexible rod 140. The flexible rod 140 is driven by the linear motions of the drive sub assembly which translates the linear motion ultimately to the needle bundle 152. An end 137 of the flexible rod 140 interfaces with features within the handpiece 117 that translate linear motion to the cartridge assembly 101 and the needle bundle 152.

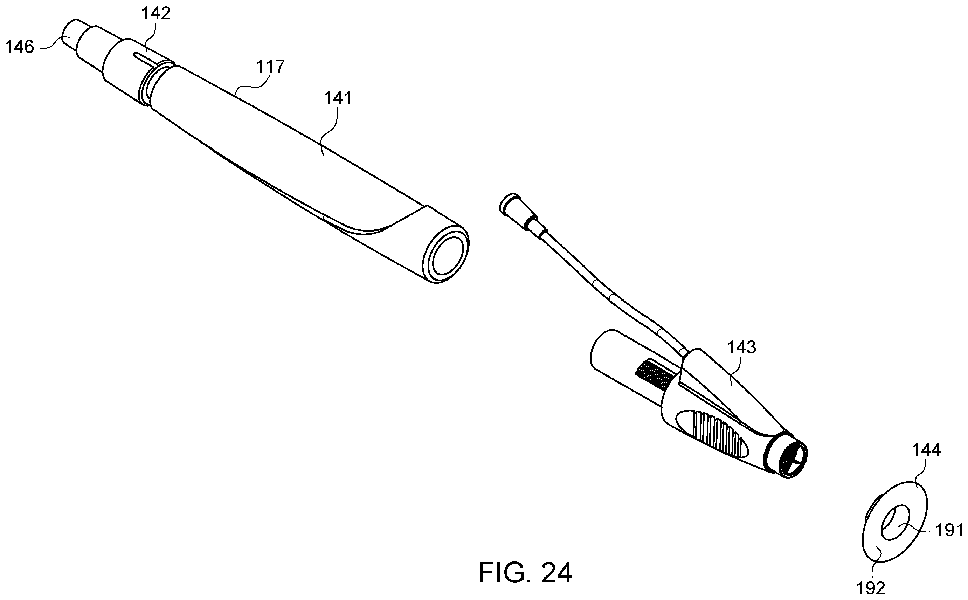

Embodiments of the handpiece 117 and cartridge143 are shown in FIG. 24. The handpiece 117 may comprise a grip or housing 141 with a collar 142 that functions as an adjuster on the end of cartridge 143 and a spacer ring 144. The handpiece sub assembly may comprise interface components 146 that interface with the flexible connection shaft for the translation of linear, and rotational motion to the cartridge 143 and the needle bundle 152.

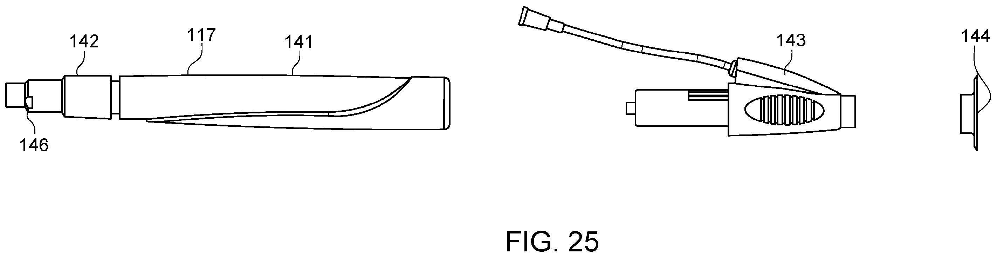

As illustrated in FIGS. 25-26, the rotational interface feature 146 at the proximal end of the handpiece 117 may accept rotational motion from the flexible connection shaft. The rotational interface feature 146 can rotate freely within the housing 141 of the handpiece 117. A bearing 147 may allow the sliding action of linear motion whilst maintaining interlock for control of rotational motion. This is where a mixing of rotational and linear motion may be performed within the handpiece 117.

In further embodiments, control of distance of linear stroke may be accomplished in the handpiece 117 instead of the drive assembly 115. Accordingly, the collar 142 may comprise a threaded feature 148 that interfaces with the housing 141 of the handpiece 117. This threaded feature may comprise stops on its rotational adjustment to prevent the collar from unthreading from the handpiece housing. The collar 142 may comprise marks that indicate coarse adjustment of the depth of needle impingement at the treatment site. Embodiments of the system may use either one or both adjustment features. Alternatively, the collar 142 may be a slider collar with detents indicating depth of needle impingement. The handpiece interface 145 may comprise a female interface feature 189 that engages a male feature 190 of the cartridge 143 and translates the motion to the needle bundle 152 within the cartridge 143.

As illustrated in FIGS. 24-26, the cartridge 143 may comprise a spacer ring 144 that is generally "hat-shaped," and has an aperture 191 that allows it to be placed on the end of the cartridge 143 and remain during the vibration that occurs during the treatment process. The spacer ring 144 may further comprise a disc 192 that can be places on or adjacent to a treatment site. The spacer ring 144 may be made from a clear plastic material and treatments may be done with or without the spacer ring 144 in place. The spacer ring 144 may provide a visual spacing cue and may help the technician space the tegula one from another to allow the skin bridge remaining between tegulae to remain and promote correct healing.

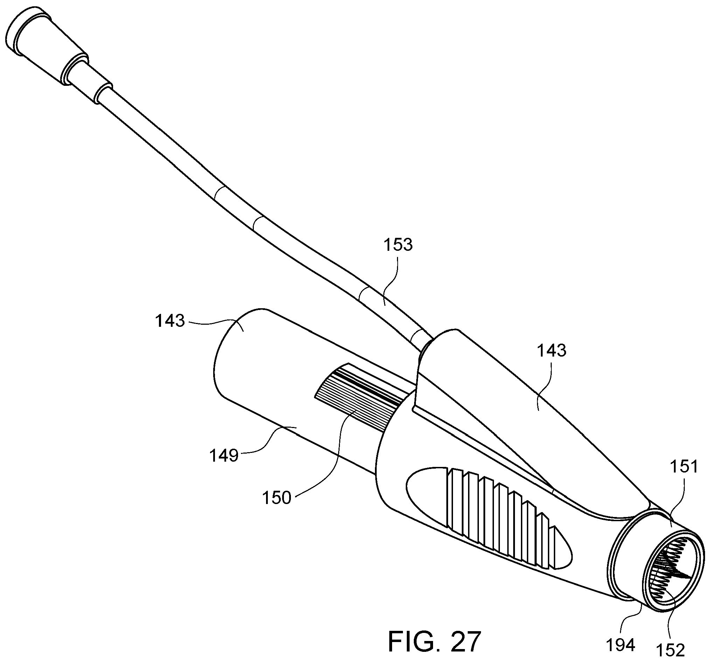

As illustrated in FIGS. 27-28, the cartridge 143 may comprise a multi-part plastic molded assembly. In accordance with embodiments of the system, a proximal end 193 of the cartridge housing 149 is cylindrical in form and engages in a press or other fit with the handpiece 117 to maintain correct interface and prevent unwanted friction of the motion drive. This cylindrical portion 193 of the cartridge housing 149 may comprise two ramped obround features 150 extending from the cylinder body. These features allow the cartridge housing 149 to be rotated and locked into place in the proximal end of the handpiece 117 that is also obround in shape. Once locked in place, the vibration that occurs during use will not be sufficient to cause these parts to separate during the treatment.

The cartridge 143 may also comprise a cylindrical feature 151 on the distal end that is the location for the attachment of the spacer ring 144. Tolerances of this feature and ring together allow the two to be mated and remain throughout the treatment cycle. On the top of the cartridge 143 is a flexible tubing set 153 that may be short as shown with a luer lock feature attached or be long and have the luer lock feature spaced a distance from the cartridge, preferably several feet away from the cartridge. In a further embodiment, the cartridge 143 may have the luer lock feature molded onto the cartridge where the tubing is attached, thereby eliminating the tubing assembly.

Within the distal end of the cartridge 144, the needle bundle 152 is seated within and protected from damage by the cylindrical flange 194 surrounding the needle bundle 152. Within the cartridge housing 149, a fluid passage 155 interfaces with the flexible tubing set 153. Through the flexible tubing set 153 and the fluid passage 155, Teprsol or other facilitating fluid may be administered to the treatment site during tattoo removal. An integral aperture may be added to the fluid passage 155 to limit the flow of the Teprsol fluid during operation. This may prevent the free flow of the fluid onto and around the treatment site possibly wasting fluid or allowing too much fluid to be released during treatment. Also, the cartridge housing 149 may comprise a spring 154 that ensures the needle bundle 152 retracts during the treatment process. The retraction may ensure that the needle bundle 152 can rotate freely during the treatment. Rotation of the needle bundle 152 may occur before total retraction of the needle bundle 152. Alternatively, the cartridge 144 may not comprise a spring, or the spring 154 may serve only to ensure that the assembly internal components are maintained in a proper position. In such embodiments the drive mechanism, including the needle interface rod 156, may directly drive the needle bundle 152 in extension and retraction such that the reciprocating motion does not depend on the action or return force of the spring 154.

A seal 159 may reduce or prevents excess Teprsol fluid from exiting to the back of the cartridge. Any excess fluid that enters this portion may exit the chamber 195 through a hole 160. At the proximal end of the cartridge 143, needle interface rod 156 provides interface with handpiece components for linear as well as rotational motion. When the handpiece parts travel in a linear motion that motion directly moves the interface rod 156 in a linear travel, thus moving the needle bundle rod 157 in a linear travel. When placed against the skin of the client, the needle bundle 152 when acted upon in a linear motion through the interface rod 156 and needle bundle rod 157, will extend from the distal end of the cartridge and pierce the skin of the client.

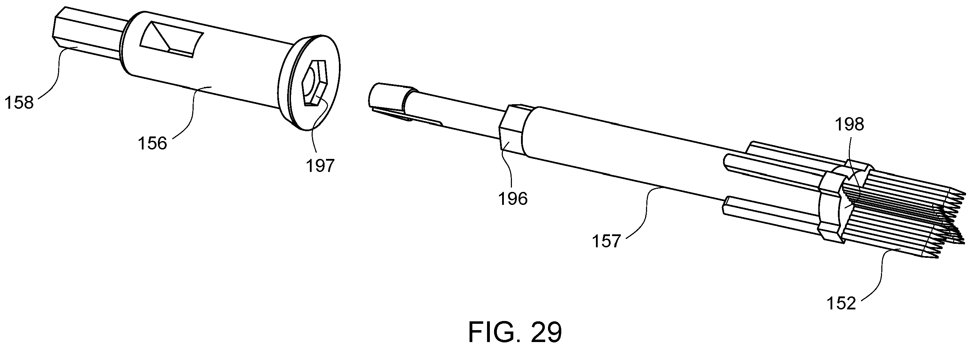

As illustrated in FIG. 29, the needle bundle 152 may be attached to a forward face 198 of the needle bundle rod 157. The needle bundle may be sized larger in diameter or smaller with high density of needles or low density of needles being spaced closely or sparsely. The needle face 198 may be designed in other patterns other than circular, such as triangular mount or square mount with various needle patterns on them along with various densities of needles. The embodiment of FIG. 29 shows the needle bundle 152 in a cross pattern. Other patterns would be understood by those familiar with the art.

The needle rod 157 supports the needle bundle 152 and provides the interface of the needle bundle to the drive components along with strength to press needles into the skin of the client. The rod may be molded in a plastic material and the needles may be machine inserted and molded in place or hand inserted and molded in place. For sparse needle configurations as described above, the needle face 198 may comprise a pattern of holes and the needles hand placed and secured with adhesive. The needle rod 157 interfaces with the interface rod 156. The two parts are assembled within the housing of the cartridge and are secured by a snap fit. The rear of the needle rod has a male hexagonal feature 196 that engages a corresponding female hexagonal feature 197 formed in interface rod 156 such that the two parts when assembled rotate as one part. The interface rod 156 may comprise an interface feature158 that engages with the handpiece interface 145. The hexagonal features ensures that rotational motion will be translated. It will be understood that other feature shapes may be used, including square, triangle, rectangle, obround, or other appropriate shapes.

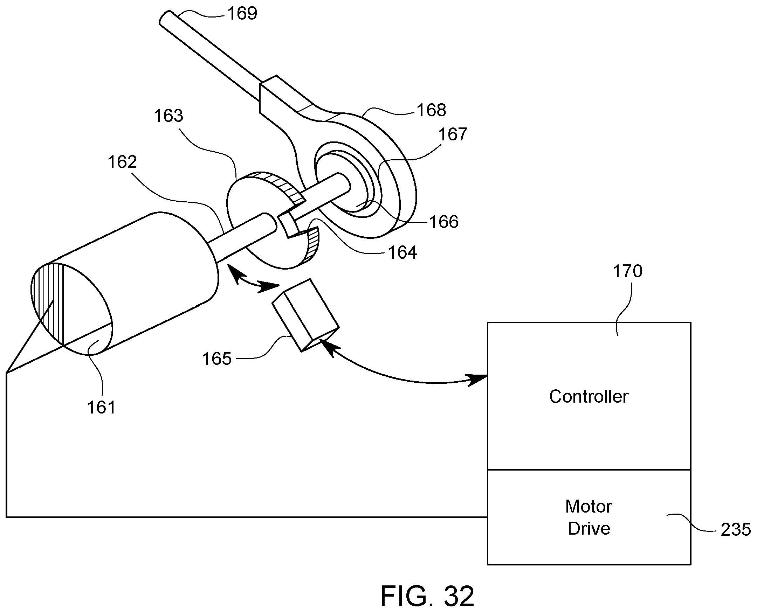

As illustrated in FIG. 32, embodiments of an oscillation motor 161may comprise a shaft 162 that rotates when voltage is applied to the motor from a drive section 235 of a controller 170. The drive section 235 of the controller 170 may apply a high voltage or long duty cycle to the motor to make it attain the speeds necessary for the treatment parameters contained within controller memory. The shaft 162 is connected to a crank 166, to which a bearing 167 allows a wire drive 168 to maintain its aligned position for driving a flexible rod 169. The shaft may comprise a disk 163 with a slot 164 or multiplicity of slots. The location of the slot 164 or features is sensed by a sensor 165 which may be optical, a magnetic device or a physical interface such as a cam follower. The output of the sensor 165 is sent to the controller 170. The controller with this information may coordinate the motion of the needle rotation in synchronization with the oscillation. This information may also be used for retracting the needle bundle upon completion of a treatment or during treatment pauses for repositioning the cartridge to another treatment site. Rotation of the needle bundle may be performed in a predetermined manner based upon treatment parameters or may be used to control the force of the rotation as a method of enhancing the treatment. Accordingly, additional abrasion may shorten the treatment time and provide the same tissue effect in tattoo removal as when the operator manually moved the needles during the treatment.

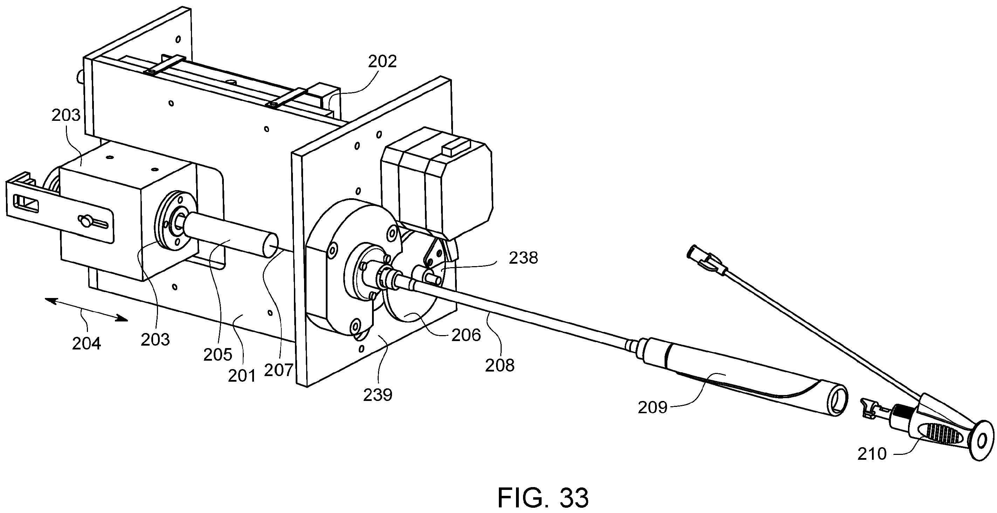

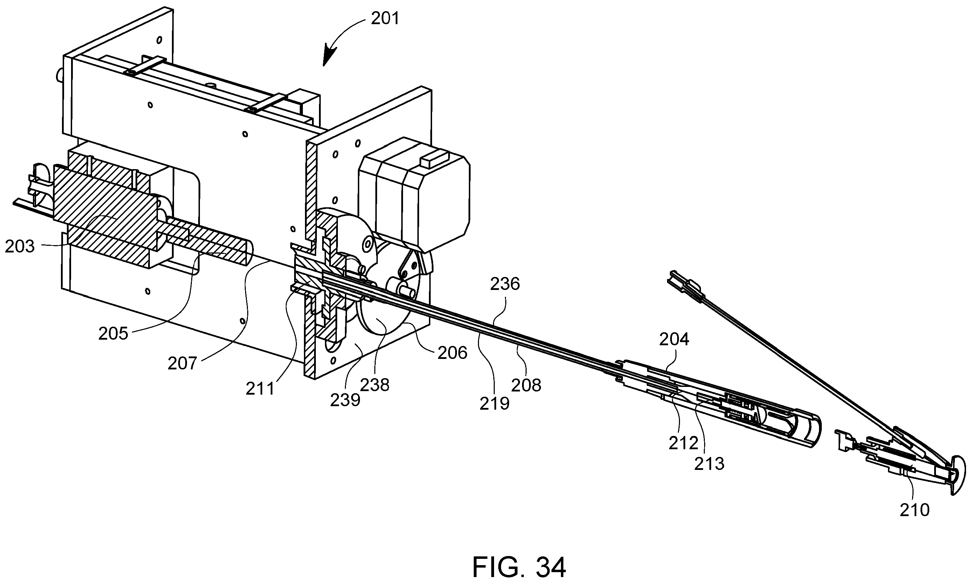

As shown in FIGS. 33-34, in some embodiments, a drive system 201 may comprise a flexible shaft 208, a handpiece 209 couplable to a disposable cartridge 210, and a drive motor 203 that may be rotated to be in alignment with a center axis of the flexible shaft 208 and coupled to a central drive wire 207 located in the center of the flexible shaft 208 via a coupling 205. The drive motor 203 may further be coupled to a movable carriage 202 on a far side of the drive assembly 201, allowing the drive motor 203 to be moved as shown along a linear axis 204 towards or away from the flexible shaft 208. The linear axis 204 allows the drive wire 207 to change position relative to the distal end of the flexible shaft 208. Coupling the drive motor 203 rotational coupling 205 to the drive wire 207 causes the drive wire to rotate. A flexible sheath or inner housing 219 surrounds the drive wire 207 is rotated by a stepper motor 238 and a gear combination 206 located on a face 239 of the drive system 201. The gear combination thereby translates rotational motion into the flexible shaft using a gear coupling 211.

Embodiments of the flexible shaft 208 comprise an outer sheath 236, an inner sheath 219, and the drive wire 207. The inner sheath 219 may comprise a rotational coupling 212 on a proximal end, located within the handpiece 209 that is driven by the gear coupling 211. The gear coupling 211 translates rotational motion to the inner sheath from a first end to a second end of the flexible shaft 208. The drive wire 207 is coupled to the drive motor 203 by the coupling 205. As connected in this fashion, the drive wire 207 may be rotated at any speed by the drive motor 203 translating that rotational motion to the rotational coupling 212 located within the handpiece 209. The drive wire 207 and the inner sheath 219 may rotate independently. The rotation of the inner sheath 219 in the flexible shaft 208 may be independently driven by the gear combination 206. Accordingly, all three motions are translated to the flexible shaft 208 and transferred to other inner components located within the handpiece 209.

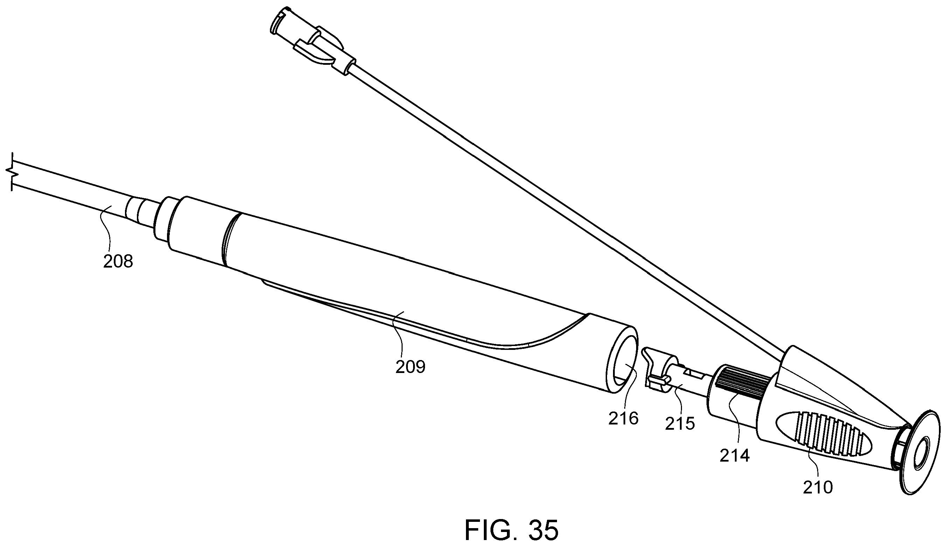

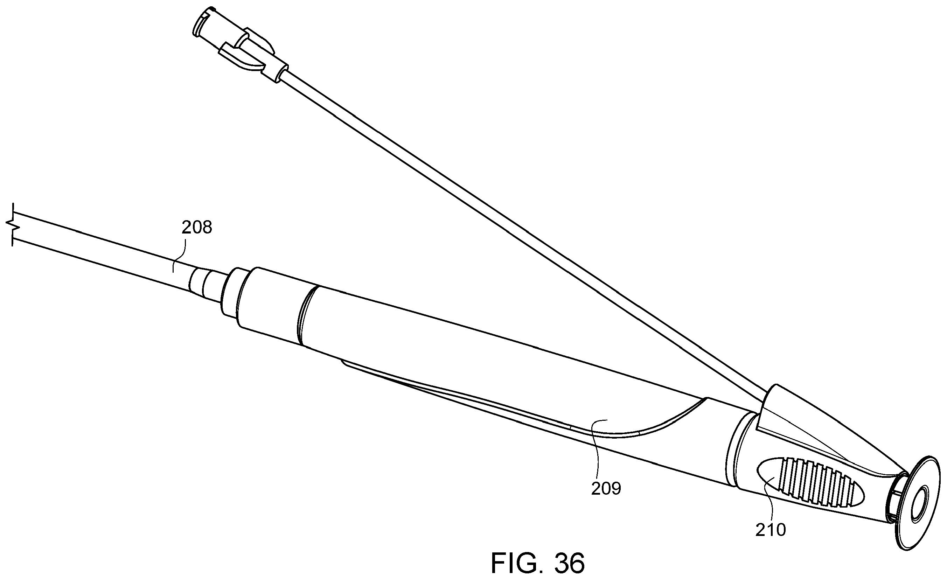

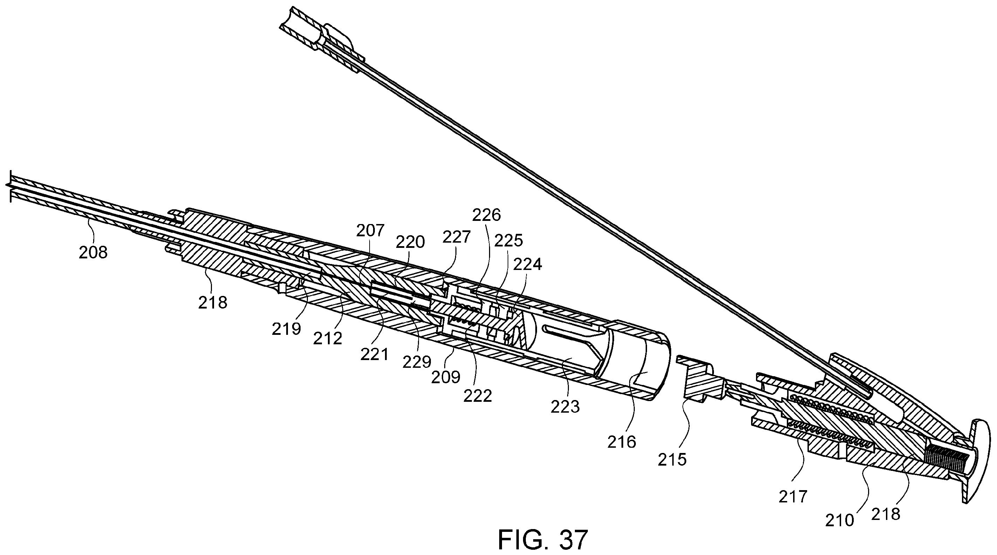

As illustrated in FIGS. 35-37, the disposable cartridge 210 may comprise a follower 215 and locking ridges 214 positioned on a distal end of the disposable cartridge 210. The handpiece 209 may also comprise a recess 216 on a proximal end that receives the locking ridges 214 on the distal end of the disposable cartridge 210. Once inserted into the recess 216, the disposable cartridge 210 may be rotated slightly to secure and lock the handpiece 209 and the disposable cartridge 210 together as one single unit.

The flexible shaft 208 is couplable to a needle bundle 218 which in turn can be attached or removed from the handpiece 209. At the distal end of the handpiece 209, the inner sheath 219 may terminate into the rotational coupling 212. The drive wire 207 may likewise terminate and be secured in a coupler 220. The drive wire may be inserted into a recess 221 in a proximal end of the coupler 220. When the drive wire 207 is rotated at the drive motor 203 this motion is translated to the coupler 220. It will be appreciated that the design of the drive system 201 allows the service and assembly of the handpiece 209 or the flexible shaft 208 independently of one another, while still being able to translate rotational motion into linear motion once assembled.

Within the handpiece 209, the coupler 220 may be coupled to a swashplate 224. The swashplate 224 may be manufactured from a hardened material that is resistant to wear through use, such as a hardened polished steel or similar metals or alloys. Also, the swashplate 224 may comprise a sliding receptacle 229 that mates with the coupler 220 and transfers the rotational motion from the drive wire 207 to the rear of the swashplate 224. The rotational coupling 212 may be coupled to a bearing retainer 227 and a rotational housing 223. The rotational motion from the flexible shaft 208 is then transferred from the coupler 220 to the swashplate 224 and the rotational motion is transferred from the rotational coupling 212 coupled to the rotational housing 223 through the bearing retainer 227.

To ensure that the rotation motion from the drive wire 207 is translated with minimal friction, the swashplate 224 rotates within a first radial bearing 225 and a second radial bearing 226. It will be appreciated that this rotation ensures that accurate and free motion is available to operate the drive system 201. The first and second bearings 225, 226 may be preloaded to ensure proper rotational freedom by a second compression spring 222. The first and second bearings 225, 226 are retained by the bearing retainer 227, which may be a separate piece to allow installation of the first and second bearings 225, 226 during the initial assembly of the drive system 201. After assembly, the bearing retainer 227 may be coupled to the rotational housing 223, ensuring that the rotational components are both secure and operate freely for motion translation.

The follower 215 may be rigidly coupled to the needle bundle 218. The handpiece 209 may comprise a compression spring 217 that pushes the follower 215 away from the disposable cartridge 210 and maintains the needle bundle 218 fully retracted within the disposable cartridge 210. The compression spring 217 may be positioned as shown to maintain the follower 215 in a fixed, initial position until the follower 215 is pushed toward the disposable cartridge 210 causing the compression spring 217 to become compressed. When released, the follower 215 will naturally return to the initial position at rest, fully retracting the needle bundle 218 into the disposable cartridge 210. Alternatively, the handpiece may not comprise a spring, or the spring 217 may serve only to ensure that the assembly internal components are maintained in a proper position. In such embodiments the drive mechanism, including the drive wire 207, may directly drive the needle bundle 218 in extension and retraction such that the reciprocating motion does not depend on the action or return force of the spring 217.

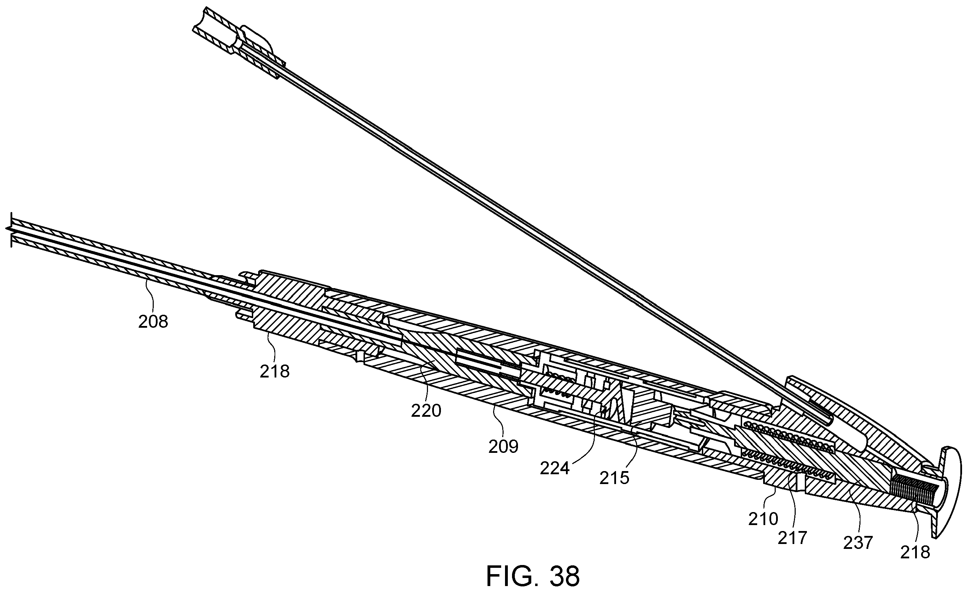

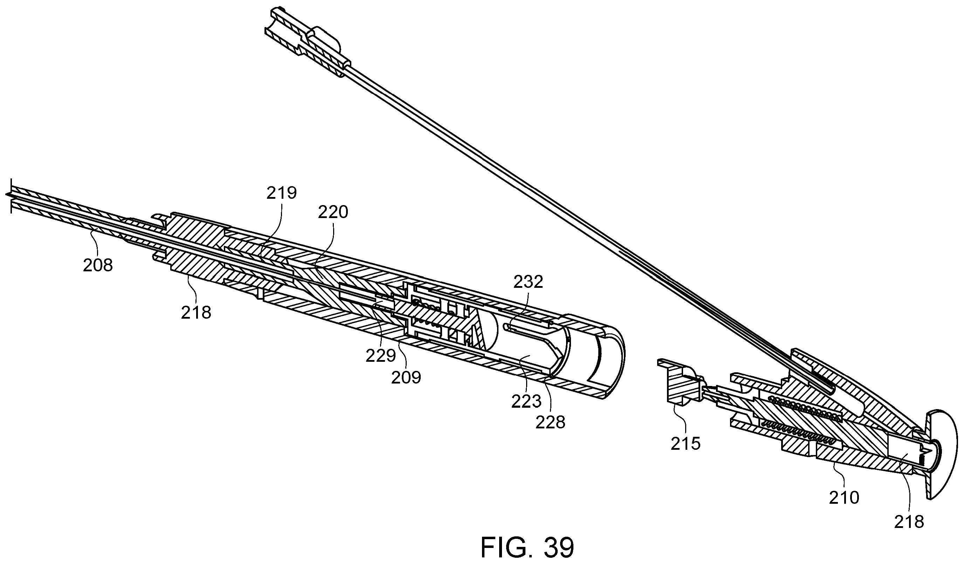

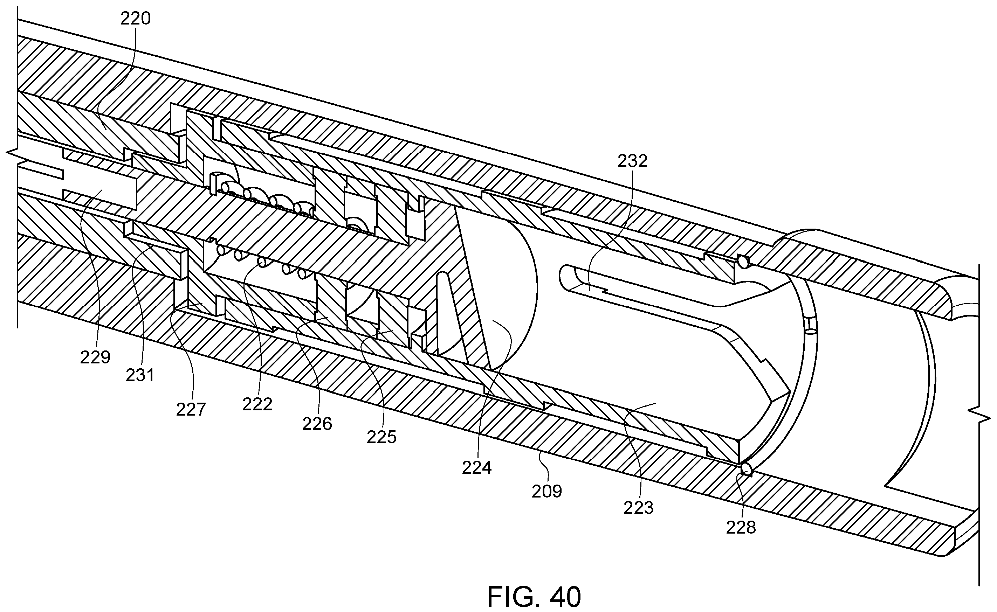

As illustrated in FIGS. 38-40, embodiments of the disposable cartridge 210 may be coupled to the handpiece 209. When coupled together, the follower 215 impinges on the swashplate 224. As the swashplate 224 is rotated, the follower 215 is pushed from an initial position at rest, thereby compressing the compression spring 217 and causing the needle bundle 218 to also move forward and be exposed from within a housing of the disposable cartridge 210. Next, as the swashplate is rotated fully, the follower 215 engages the surface feature of the swashplate 224, causing a displacement of the follower 215 from one extreme of the swashplate 224 profile to the other. This rotational motion of the swashplate 224 translates the motion of the follower 215 into linear motion, causing the needle interface rod 237 and needle bundle 218 to either extend or retract. The conversion of linear motion allows the needle bundle 218 to impinge the patient's skin surface to perform the linear needle motion during the abrasion process. Therefore, the rotational motion from the drive wire 207 is translated into linear motion at the needle bundle 218.

A coupling feature 231 of the bearing retainer 227 mates with the coupler 220 that is driven by the inner sheath 219 in the flexible shaft 208. Because the bearing retainer 227 and the rotational housing 223 are coupled to one another, when the inner sheath 219 is driven in a rotational fashion by the gear combination 206 of the drive system 201, the rotational motion is transferred to the coupler 220 which in turn rotates the bearing retainer 227 and the rotational housing assembly 223. The rotational housing 223 is free to rotate within the handpiece housing 209 and to prevent its departure from the housing 209 is retained by a retainer ring 228.

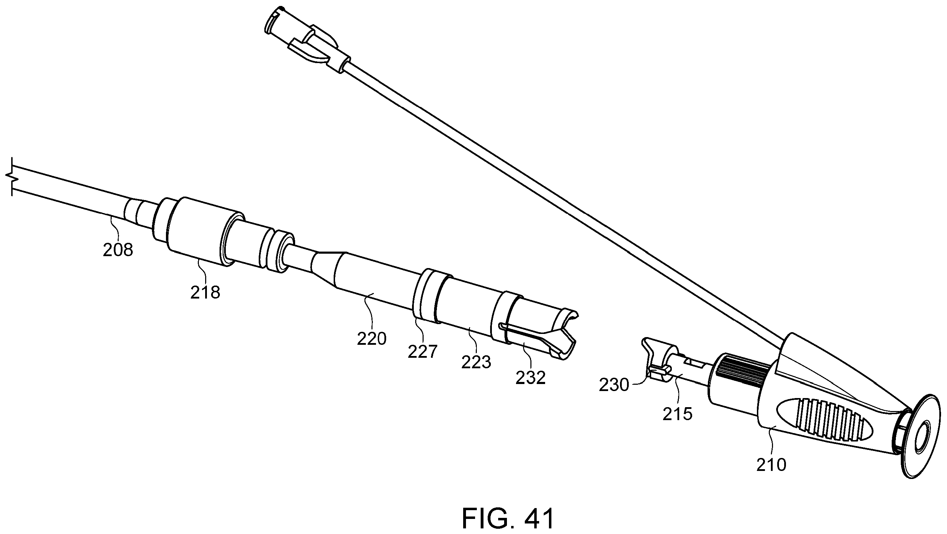

As illustrated in FIG. 41, the coupler 220 may rotate the bearing retainer 227 and the rotational housing 223. The rotational housing 223 may comprise slots 232. As the rotational housing 223 rotates within the handpiece 209, the slots 232 and anything within the slots 232 will rotate. The follower 215 may comprise tabs 230 that are comparable in size and width to fit within the slots 232 on the rotational housing 223.

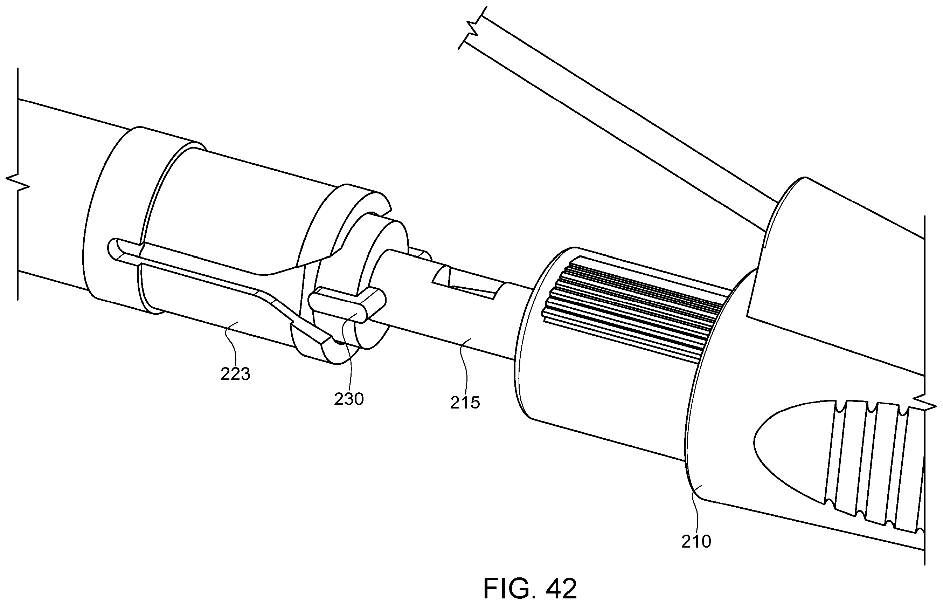

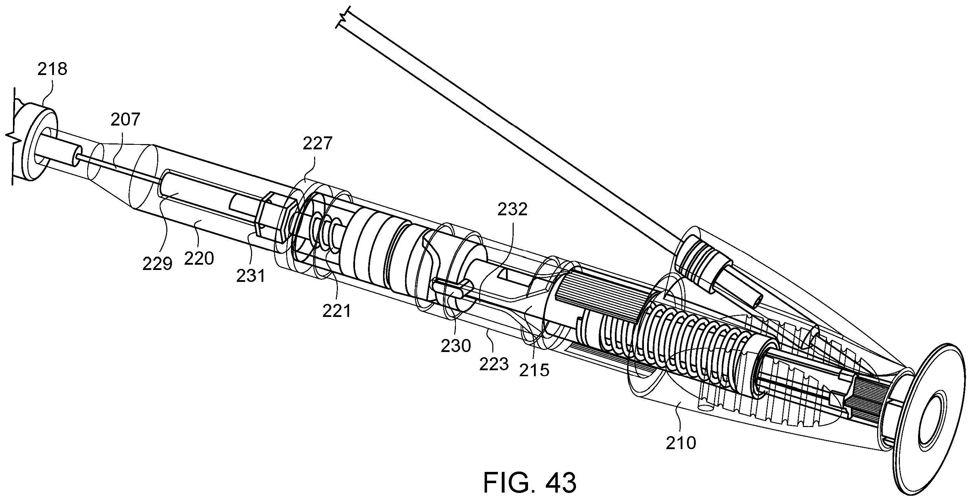

As illustrated in FIGS. 42-43, the follower 215 may be aligned for assembly with the disposable cartridge 210 such that the tabs 230 on the follower 215 are inserted into the slots 232 on the rotational housing 223. In embodiments of the system, the rotational coupler 223 rotates independently of the handpiece housing 209, and the follower 215 located in the disposable cartridge 210 and coupled to the needle bundle 218 also rotates, being driven by the tabs 230 and slots 232 of the rotational housing 223. Thus, it will be appreciated that in some embodiments, the linear motion of the needle bundle 218 can be performed via the swashplate 224 being rotated and the follower 224 being driven by that motion. The rotational motion of the follower 215 may be simultaneously performed via the rotation of the rotational housing 223, independent of the handpiece housing 209.

In tattoo removal, it is desirable to vary the depth that the needles can operate during the abrasion process. Tattoo ink depth varies from one patient to another and there is no set distance for ink depth since all skin physiology varies across the board from patient to patient. Thus, embodiments of the device allow needle penetration to be varied to accommodate patients of all physiologies. The depth may be varied and controlled to ensure the depth is known and repeatable or can be counted on to be accurate. The carriage 202 is moveable and accurately positioned allowing the drive motor 203 to be positioned relative to the handpiece 209. Since the drive wire 207 is rigid and moves freely within the flexible shaft 208, any position of the drive motor 203 in the drive assembly 201 will translate into the end of the drive wire 207 being moved or positioned as desired withing the handpiece 209. To vary the position of the needle bundle 218 in the disposable cartridge 210, a method is employed to move the needles by moving the impinging features of the swashplate 224 and follower 215. In this case the wire drive coupler is moved by the drive wire being repositioned.

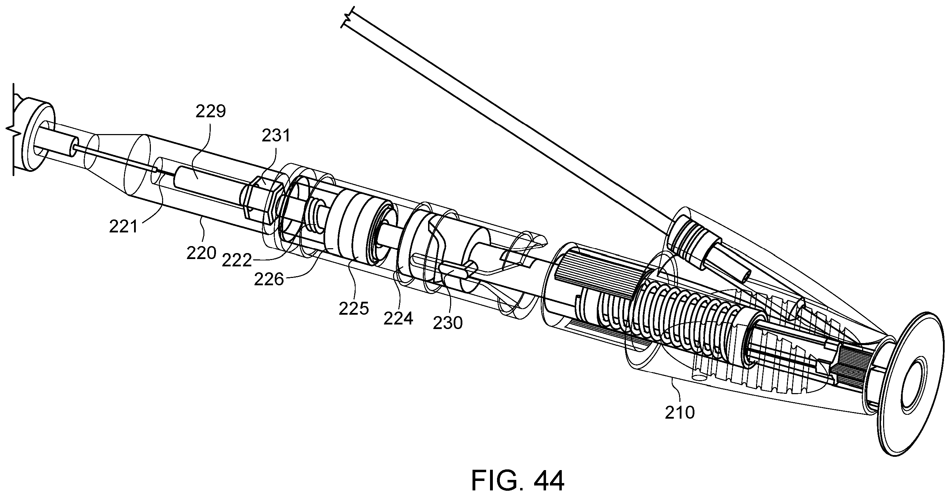

As illustrated in FIG. 44, embodiments of the system comprise a rotational shaft that may be moved forward toward a proximal end of the disposable cartridge 210 causing displacement of the needle bundle 218. The repositioning process pushes on the distal end of the swashplate 224, pushing it away from the bearings, which repositions the face of the swashplate 224, thus pushing the follower into the disposable cartridge 210 and thus repositioning the needle bundle 218. With these items repositioned, due to the drive motor 203 position, in the drive assembly 201, the depth of the needle bundle 218 may be controlled during its stroke and rotation. The compression spring 217 located in the distal end of the rotational housing 223 and the bearing assembly 227 also acts to retract the swashplate 224 when returning to the initial position at rest. It will be appreciated that the drive assembly 201 enables the motion of the disposable cartridge 210 and, ultimately in the tattoo removal process, needle linear motion for abrasion, needle rotational position or rotational motion enhancing the abrasion, and needle depth control and position relative to the surface of the patient's skin. In some embodiments, the swashplate 224 may be incorporated into a condensed version that would allow the drive motor 203 to be housed within the handpiece and perform the functions as described above.

## Claims

1. A system for use in the application and removal of tattoos and other skin treatments, comprising:
a handpiece (2, 117, 209);
a disposable cartridge (37, 143, 210) couplable to the handpiece, the disposable cartridge comprising a needle bundle (49, 152, 218) and a flexible tubing connection (54, 153) couplable to a fluid pump (13) configured to dispense facilitating fluid to the needle bundle;
a system controller (3, 105) comprising a power input (6) , a microprocessor (7), and a graphics processing unit (12), the system controller configured to control the operation of one or more drives;
an oscillation drive (85) comprising an oscillation drive frame (50), an oscillation motor (22, 124), and an eccentric crank (28, 118), the oscillation drive configured to provide oscillatory motion to the needle bundle;
a depth drive (83) comprising a block (21), a first motor (23, 119), a gear train (24), a lead screw shaft (25, 128), and a senor, the depth drive configured to provide linear motion to the needle bundle;
a rotational drive (89) comprising a motor (32), a drive rod (30), and a gear combination (36), the rotational drive configured to provide rotational motion to the needle bundle; and
a drive shaft (38, 156) configured to independently translate oscillatory, linear, and rotational motion to the needle bundle.

2. The system of claim 1, further comprising a wearable wrist controller (5, 107) configured to adjust treatment parameters of the handpiece.

3. The system of claim 1 or 2, further comprising a foot controller (106) configured to start and stop operation of the system when the foot controller is engaged and disengaged.

4. The system of any preceding claim, further comprising a tablet controller.

5. The system of any preceding claim, wherein the system controller is configured to control the initiation, flow rate, and stoppage of the facilitating fluid dispensed by the fluid pump.

6. The system of any preceding claim, wherein the needle bundle comprises a plurality of blades (71) having an angled configuration, the plurality of blades oriented at varying heights along a linear axis.

7. The system of any one of claims 1 to 5, wherein the plurality of blades is configured to allow even abrasion when rotated and oscillated at the same time.

8. The system of any preceding claim, wherein the drive shaft is flexible.

9. The system of any of claims 1-5, wherein the needle bundle comprises a compact linear configuration (63), the needle bundle configured to orient a group of needles in a single layered arrangement.

10. The system of any of claims 1-5, wherein the needle bundle comprises a spaced linear configuration (64), the needle bundle configured to orient a group of needles spaced apart from one another without touching or overlapping needles.

11. The system of any of claims 1-5, wherein the needle bundle comprises a staggered configuration (65), the needle bundle configured to orient a group of needles in two or more rows with the needles in a first row offset spatially from the needles in a second row.

12. The system of any preceding claim, wherein the microprocessor is configured to operate the oscillation drive, the distance drive, and the rotational drive individually and cooperatively.

13. The system of any preceding claim, wherein the needle bundle retracts into a housing of the disposable cartridge when the oscillation drive is paused or stopped.

14. The system of any preceding claim, further comprising a substantially hat-shaped spacer ring (144) coupled to the disposable cartridge configured to space the treatment sites at predetermined intervals without having to mark the skin of a patient beforehand.

## Patentansprüche

1. System für die Verwendung bei der Anfertigung und Entfernung von Tätowierungen und anderer Hautbehandlungen, umfassend:
ein Handteil (2, 117, 209);
eine an das Handteil ankoppelbare Einwegkartusche (37, 143, 210), wobei die Einwegkartusche ein Nadelbündel (49, 152, 218) und eine flexible Schlauchverbindung (54, 153) umfasst, die an eine Fluidpumpe (13) ankoppelbar ist, die so konfiguriert ist, dass sie dem Nadelbündel ein unterstützendes Fluid zuführt;
eine Systemsteuerung (3, 105), umfassend einen Stromeingang (6), einen Mikroprozessor (7) und eine Grafikprozessoreinheit (12), wobei die Systemsteuerung so konfiguriert ist, dass sie den Betrieb eines oder mehrerer Antriebe steuert;
einen Oszillationsantrieb (85), umfassend einen Rahmen (50) des Oszillationsantriebs, einen Oszillationsmotor (22, 124) und eine Exzenterkurbel (28, 118), wobei der Oszillationsantrieb so konfiguriert ist, dass er dem Nadelbündel eine oszillierende Bewegung verleiht;
einen Tiefenantrieb (83), umfassend einen Block (21), einen ersten Motor (23, 119), ein Getriebe (24), eine Leitspindelwelle (25, 128) und einen Sensor, wobei der Tiefenantrieb so konfiguriert ist, dass er dem Nadelbündel eine lineare Bewegung verleiht;
einen Rotationsantrieb (89), umfassend einen Motor (32), eine Antriebsstange (30) und eine Getriebekombination (36), wobei der Rotationsantrieb so konfiguriert ist, dass er dem Nadelbündel eine Rotationsbewegung verleiht; und
eine Antriebswelle (38, 156), die so konfiguriert ist, dass sie dem Nadelbündel unabhängig voneinander eine oszillierende, lineare und rotatorische Bewegung überträgt.

2. System nach Anspruch 1, ferner umfassend eine am Handgelenk tragbare Steuerung (5, 107), die so konfiguriert ist, dass sie Behandlungsparameter des Handteils einstellt.

3. System nach Anspruch 1 oder 2, ferner umfassend eine Fußsteuerung (106), die so konfiguriert ist, dass sie den Betrieb des Systems startet und stoppt, wenn die Fußsteuerung betätigt bzw. nicht betätigt wird.

4. System nach einem der vorstehenden Ansprüche, ferner umfassend eine Tabletsteuerung.

5. System nach einem der vorstehenden Ansprüche, wobei die Systemsteuerung so konfiguriert ist, dass sie die Initiierung, die Durchflussrate und das Stoppen der von der Fluidpumpe abgegebenen unterstützenden Flüssigkeit steuert.

6. System nach einem der vorstehenden Ansprüche, wobei das Nadelbündel eine Vielzahl von Klingen (71) mit einer abgewinkelten Konfiguration umfasst, wobei die Vielzahl von Klingen entlang einer linearen Achse in unterschiedlichen Höhen angeordnet ist.

7. System nach einem der Ansprüche 1 bis 5, wobei die Vielzahl von Klingen so konfiguriert ist, dass sie eine gleichmäßige Abrasion ermöglicht, wenn sie gleichzeitig rotiert und oszilliert wird.

8. System nach einem der vorstehenden Ansprüche, wobei die Antriebswelle flexibel ist.

9. System nach einem der Ansprüche 1 bis 5, wobei das Nadelbündel eine kompakte lineare Konfiguration (63) umfasst, wobei das Nadelbündel so konfiguriert ist, dass es eine Gruppe von Nadeln in einer einschichtigen Anordnung ausrichtet.

10. System nach einem der Ansprüche 1 bis 5, wobei das Nadelbündel eine beabstandete lineare Konfiguration (64) umfasst, wobei das Nadelbündel so konfiguriert ist, dass es eine Gruppe von Nadeln im Abstand voneinander ausrichtet, ohne dass sich Nadeln berühren oder überlappen.

11. System nach einem der Ansprüche 1 bis 5, wobei das Nadelbündel eine versetzte Konfiguration (65) umfasst, wobei das Nadelbündel so konfiguriert ist, dass es eine Gruppe von Nadeln in zwei oder mehr Reihen ausrichtet, wobei die Nadeln in einer ersten Reihe räumlich gegenüber den Nadeln in einer zweiten Reihe versetzt sind.

12. System nach einem der vorstehenden Ansprüche, wobei der Mikroprozessor so konfiguriert ist, dass er den Oszillationsantrieb, den Abstandsantrieb und den Rotationsantrieb einzeln und koordiniert betreibt.

13. System nach einem der vorstehenden Ansprüche, wobei sich das Nadelbündel in ein Gehäuse der Einwegkartusche zurückzieht, wenn der Oszillationsantrieb pausiert oder angehalten wird.

14. System nach einem der vorstehenden Ansprüche, ferner umfassend einen im Wesentlichen hut-förmigen Abstandshalterring (144), der mit der Einwegkartusche gekoppelt und so konfiguriert ist, dass er die Behandlungsstellen in vorbestimmten Abständen voneinander beabstandet, ohne dass die Haut eines Patienten zuvor markiert werden muss.

## Revendications

1. Système destiné à être utilisé pour l'application et l'enlèvement de tatouages et pour d'autres traitements de la peau, comprenant :
une pièce à main (2, 117, 209) ;
une cartouche jetable (37, 143, 210) pouvant être accouplée à la pièce à main, la cartouche jetable comprenant un faisceau d'aiguilles (49, 152, 218) et un raccord de tubulure souple (54, 153) pouvant être accouplé à une pompe à fluide (13) conçue pour distribuer un fluide facilitant au faisceau d'aiguilles.
un dispositif de commande du système (3, 105) comprenant une entrée d'alimentation (6), un microprocesseur (7) et une unité de traitement graphique (12), le dispositif de commande du système étant conçu pour commander le fonctionnement d'un ou plusieurs entraînements.
un entraînement par oscillation (85) comprenant une structure d'entraînement par oscillation (50), un moteur d'oscillation (22, 124) et une manivelle excentrique (28, 118), l'entraînement par oscillation étant conçu pour fournir un mouvement oscillatoire au faisceau d'aiguilles.
un entraînement en profondeur (83) comprenant un bloc (21), un premier moteur (23, 119), un train d'engrenages (24), un arbre de vis mère (25, 128) et un capteur, l'entraînement en profondeur conçu pour fournir un mouvement linéaire au faisceau d'aiguilles ;
un entraînement rotatif (89) comprenant un moteur (32), une tige d'entraînement (30) et une combinaison d'engrenages (36), l'entraînement rotatif étant conçu pour fournir un mouvement rotatif au faisceau d'aiguilles ; et
un arbre d'entraînement (38, 156) conçu pour translater un mouvement oscillatoire, linéaire et rotatif de manière indépendante vers le faisceau d'aiguilles.

2. Système selon la revendication 1, comprenant en outre un dispositif de commande au poignet portable (5, 107) conçu pour ajuster les paramètres de traitement de la pièce à main.

3. Système selon la revendication 1 ou 2, comprenant en outre un dispositif de commande au pied (106) conçu pour démarrer et arrêter le fonctionnement du système lorsque le dispositif de commande au pied est engagé et désengagé.

4. Système selon une quelconque revendication précédente, comprenant un dispositif de commande par tablette :

5. Système selon l'une quelconque des revendications précédentes, dans lequel le dispositif de commande du système est conçu pour commander le déclenchement, le débit et l'arrêt du fluide facilitant distribué par la pompe à fluide.

6. Système selon l'une quelconque des revendications précédentes, dans lequel le faisceau d'aiguilles comprend une pluralité de lames (71) présentant une configuration inclinée, la pluralité de lames étant orientée à des hauteurs variables le long d'un axe linéaire.

7. Système selon l'une quelconque des revendications 1 à 5, dans lequel la pluralité de lames est conçue pour permettre une abrasion uniforme lorsqu'elles sont tournées et mises en oscillation en même temps.

8. Système selon une quelconque revendication précédente, dans lequel l'arbre d'entraînement est flexible.

9. Système selon l'une quelconque des revendications 1 à 5, dans lequel le faisceau d'aiguilles comprend une configuration linéaire compacte (63), le faisceau d'aiguilles étant conçu pour orienter un groupe d'aiguilles dans un agencement à une seule couche.

10. Système selon l'une quelconque des revendications 1 à 5, dans lequel le faisceau d'aiguilles comprend une configuration linéaire espacée (64), le faisceau d'aiguilles étant conçu pour orienter un groupe d'aiguilles espacées les unes des autres sans toucher ou chevaucher les aiguilles.

11. Système selon l'une quelconque des revendications 1 à 5, dans lequel le faisceau d'aiguilles comprend une configuration en quinconce (65), le faisceau d'aiguilles étant conçu pour orienter un groupe d'aiguilles en deux rangées ou plus, les aiguilles d'une première rangée étant décalées spatialement par rapport aux aiguilles d'une seconde rangée.

12. Système selon l'une quelconque des revendications précédentes, dans lequel le microprocesseur est conçu pour actionner l'entraînement par oscillation, l'entraînement de distance et l'entraînement rotatif de manière individuelle et conjointement.

13. Système selon l'une quelconque des revendications précédentes, dans lequel le faisceau d'aiguilles se rétracte dans un logement de la cartouche jetable lorsque l'entraînement par oscillation est interrompu ou arrêté.

14. Système selon l'une quelconque des revendications précédentes, comprenant en outre une bague d'espacement sensiblement en forme de chapeau (144) accouplée à la cartouche jetable conçue pour espacer les sites de traitement à des intervalles prédéfini sans avoir à marquer au préalable la peau d'un patient.
